# EUROPEAN PATENT APPLICATION

(11) **EP 4 538 288 A1**
(43) Date of publication of application: **16.04.2025**
(21) Application number: 23819116.7
(22) Date of filing: 06.06.2023
(51) Int. Cl.: C07K 16/28, C12N 15/13, C12N 15/63, A61K 39/395, A61P 35/00

(54) **ANTI-CD40 ANTIBODY AND USE THEREOF**

(30) Priority: 06.06.2022 CN 202210629717
(71) Applicant: Biotheus Inc., Tangjiawan Town, Xiangzhou District Zhuhai, Guangdong 519080 (CN)
(72) Inventor: ZHANG, Zhenqing, Zhuhai, Guangdong 519080 (CN); JIA, Yunli, Zhuhai, Guangdong 519080 (CN); TSUN, Andy, Zhuhai, Guangdong 519080 (CN); PRINZ, Bianka, Zhuhai, Guangdong 519080 (CN); HU, Gang, Zhuhai, Guangdong 519080 (CN); LU, Qiang, Zhuhai, Guangdong 519080 (CN); BOLAND, Nadthakarn, Zhuhai, Guangdong 519080 (CN)
(74) Representative: Ghirardi, Valeria
(86) International application number: PCT/CN2023/098615
(87) International publication number: WO 2023/236951

(57) **Abstract**

The present invention relates to an antibody specifically binding to CD40 or an antigen-binding fragment thereof, and a composition containing the antibody or the antigen-binding fragment thereof. Furthermore, the present invention relates to a nucleic acid encoding the antibody or the antigen-binding fragment thereof, and a host cell comprising same, and the related use. In addition, the present invention relates to the therapeutic and detection uses of the antibodies or the antigen-binding fragments thereof.

## Description

### Technical Field

The present application relates to an antibody or antigen-binding fragment thereof that specifically binds CD40 and a composition containing the antibody or antigen-binding fragment thereof. Furthermore, the present application relates to a nucleic acid molecule encoding the antibody or antigen-binding fragment thereof and a host cell comprising the nucleic acid molecule, and related uses thereof. Furthermore, the present application relates to the therapeutic and detection uses of the antibody or antigen-binding fragment thereof.

### Background Art

CD40, also known as TNFRSF5, belongs to the tumor necrosis factor receptor superfamily (TNFRSF), which is widely expressed in diverse cell types, including immune B cells, antigen-presenting cells (APCs), and some tumor cells. The CD40 precursor contains 297 amino acids (aa) and is a type I transmembrane glycoprotein, consisting of an N-terminal signal peptide (20aa), an extracellular domain (193aa), a transmembrane domain (22aa), and a cytoplasmic domain (62aa). The extracellular domain contains four cysteine-rich domains (CRDs) and has 22 cysteines in total. CD40 is highly glycosylated, with a molecular weight calculated from the amino acid sequence of 28kD, but the glycosylated molecular weight is 40-50kD.

The ligand for CD40, CD154 (also known as TNFSF5 or CD40L) is a 34-39 kDa type II integral membrane protein. Expression of CD40L is usually inducible and restricted to cells of the hematopoietic system, such as platelets, granulocytes, activated T cells, activated B cells and activated natural killer (NK) cells, but is also weakly expressed on endothelial and smooth muscle cells.

Extensive research has confirmed that the CD40-CD40L interaction leads to the development of CD8+ cytotoxic T lymphocytes, which is crucial in immune responses and is essential for adaptive immune responses.

CD40 expression on monocytes, and their progeny macrophages and DC, and B cells plays an important role in immune cell function. Monocytes are innate immune precursor cells with very high plasticity. Monocytes have the ability to differentiate into several cell types, such as myeloid-derived suppressor cells (MDSC), macrophages, and DCs. CD40 signaling is an important trigger of the monocyte maturation process and mainly drives differentiation into macrophages of the M1 spectrum and DC. CD40 engagement on the surface of DC promotes cytokine and chemokine production, induces expression of costimulatory molecules, and facilitates the cross-presentation of antigens. One of the main functions of CD40L is to enhance antigen-presentation to T cells by activating DC. This step, called "licensing", increases the interaction of DC with T cells by upregulation of surface proteins such as CD54 and CD86, and thus activates the latter.

B cells are likewise targets of CD40L activity. The interaction between B cells and activated T cells expressing CD40L also increases the expression of MHC-II and costimulatory molecules like CD80 or CD86 and induces Ig-class switching in B cells. Activated B cells migrate to lymphoid organs where they present antigen to T cells, and CD40-activated DC and B cells support the immune response by releasing immunostimulatory cytokines and chemokines like IL-12p70, CXCL10, and TNFα. In addition, CD40-activated B cells are able to prime antigen-specific CD8+ T cells with enhanced secretion of cytokines including TNFα and IFNγ. Several studies provided evidence that ex vivo-activated CD40-expressing B cells are fully functional antigen-presenting B cells and subsequent adoptive cell transfer (ACT) therapy with these cells improved anti-tumor efficacy.

CD40 plays a pivotal role in anti-tumor immune responses, and various strategies to induce CD40 signaling have been extensively researched and explored. Current drug development targeting CD40 can be categorized into agonists (activators) or antagonists (inhibitors), depending on whether the goal is to enhance immune activity in tumor microenvironment or to suppress the immune system in autoimmune disease. However, as a monotherapy, CD40 antibodies has only demonstrated modest activity, with tumor objective response rate (ORR) < 20%. This is particularly the case in "cold tumors" where the immune response is insufficient or in tumors where immune cells have not yet infiltrated extensively. As a result, many CD40 monoclonal antibody projects have been halted due to these limitations.

Given that the significance of CD40 in cancer immunity, CD40 is considered an extremely attractive target for cancer immunotherapy. There is a strong demand in the field for the development of new anti-CD40 antibodies, especially those targeting different epitopes, capable of both blocking and non-blocking the binding of the ligand CD40L. These antibodies have varying potential applications in tumor immunity and autoimmune diseases, and when combined with clinical combination therapies, can be used for disease treatment, particularly for cancer therapy.

### Contents of the the present application

After extensive research, the inventors of the present application screened and obtained a series of fully human antibodies against CD40, which have high binding affinity to CD40, exhibit cross-reactivity with human and cynomolgus CD40, and can either effectively block or non-block the binding of CD40 to its ligand CD40L. Based on this, the present application also provides a composition containing the antibody or antigen-binding fragment thereof, a nucleic acid molecule encoding the antibody or antigen-binding fragment thereof, a host cell containing the same, and related uses thereof.

### Antibody or antigen-binding fragment

Therefore, in a aspect, the present application provides an antibody or antigen-binding fragment thereof capable of specifically binding CD40, the antibody or antigen-binding fragment thereof comprises:
(1) the following three complementary determining regions (CDR) of a heavy chain variable region (VH):
   (a) a VH CDR1, having a structure selected from the group consisting of:
      FTFX₁X₂YX₃MH (SEQ ID NO: 76), GSISSYYWS (SEQ ID NO: 6);
   (b) a VH CDR2, having a structure selected from the group consisting of:
      VIX₄YX₅X₆X₇X₈KYYAX₉SVKG (SEQ ID NO: 77), GISWSSX₁₀SIX₁₁YADSVKG (SEQ ID NO: 78), SIYYSGSTNYNPSLKS (SEQ ID NO: 22);
   (c) a VH CDR3, having a structure selected from the group consisting of:
      ARDTSRGHDI (SEQ ID NO: 23), AKDPTATTGARGYFDL (SEQ ID NO: 24), ARDANYYKWHPY (SEQ ID NO: 25);
      and/or,
(2) the following three complementary determining regions (CDR) of a light chain variable region (VL):
   (d) a VL CDR1 ,having a structure selected from the group consisting of:
      X₁₂ASQSVSX₁₃X₁₄YLA (SEQ ID NO: 79), X₁₅ASX₁₆X₁₇ISSWLA (SEQ ID NO: 7);
   (e) a VL CDR2, having a structure selected from the group consisting of:
      GASX₁₈X₁₉X₂₀X₂₁ (SEQ ID NO: 8), AASX₂₂LX₂₃S (SEQ ID NO: 9);
   (f) a VL CDR3, having a structure selected from the group consisting of:
      X₂₄X₂₅YX₂₆DYPPFT (SEQ ID NO: 10), QQX₂₇X₂₈SX₂₉PPT (SEQ ID NO: 11);
   wherein,
   X₁ is selected from the group consisting of (i) amino acid residues S, E, G, D and (ii) amino acid residues that are conservative substitutions relative to (i);
   X₂ is selected from the group consisting of (i) amino acid residues S, K, D, T and (ii) amino acid residues that are conservative substitutions relative to (i);
   X₃ is selected from the group consisting of (i) amino acid residue G, A and (ii) amino acid residues that are conservative substitutions relative to (i);
   X₄ is selected from the group consisting of (i) amino acid residues S, H and (ii) amino acid residues that are conservative substitutions relative to (i);
   X₅ is selected from the group consisting of (i) amino acid residues E, H and (ii) amino acid residues that are conservative substitutions relative to (i);
   X₆ is selected from the group consisting of (i) amino acid residue G, A and (ii) amino acid residues that are conservative substitutions relative to (i);
   X₇ is selected from the group consisting of (i) amino acid residues S, E, T, V and (ii) amino acid residues that are conservative substitutions relative to (i);
   X₈ is selected from the group consisting of (i) amino acid residues N, S, I, K and (ii) amino acid residues that are conservative substitutions relative to (i);
   X₉ is selected from the group consisting of (i) amino acid residues D, E and (ii) amino acid residues that are conservative substitutions relative to (i);
   X₁₀ is selected from the group consisting of (i) amino acid residues G, D and (ii) amino acid residues that are conservative substitutions relative to (i);
   X₁₁ is selected from the group consisting of (i) amino acid residues G, H and (ii) amino acid residues that are conservative substitutions relative to (i);
   X₁₂ is selected from the group consisting of (i) amino acid residues R, Q and (ii) amino acid residues that are conservative substitutions relative to (i);
   X₁₃ is selected from the group consisting of (i) amino acid residues Y, S and (ii) amino acid residues that are conservative substitutions relative to (i);
   X₁₄ is selected from the group consisting of (i) amino acid residues S, D, N and (ii) amino acid residues that are conservative substitutions relative to (i);
   X₁₅ is selected from the group consisting of (i) amino acid residues R, E and (ii) amino acid residues that are conservative substitutions relative to (i);
   X₁₆ is selected from the group consisting of (i) amino acid residues Q, K and (ii) amino acid residues that are conservative substitutions relative to (i);
   X₁₇ is selected from the group consisting of (i) amino acid residues G, V and (ii) amino acid residues that are conservative substitutions relative to (i);
   X₁₈ is selected from the group consisting of (i) amino acid residues S, T, A and (ii) amino acid residues that are conservative substitutions relative to (i);
   X₁₉ is selected from the group consisting of (i) amino acid residues R, L and (ii) amino acid residues that are conservative substitutions relative to (i);
   X₂₀ is selected from the group consisting of (i) amino acid residues N, A and (ii) amino acid residues that are conservative substitutions relative to (i);
   X₂₁ is selected from the group consisting of (i) amino acid residues T, N and (ii) amino acid residues that are conservative substitutions relative to (i);
   X₂₂ is selected from the group consisting of (i) amino acid residue S, Y and (ii) amino acid residues that are conservative substitutions relative to (i);
   X₂₃ is selected from the group consisting of (i) amino acid residue E, Q and (ii) amino acid residues that are conservative substitutions relative to (i);
   X₂₄ is selected from the group consisting of (i) amino acid residues S, G, Q and (ii) amino acid residues that are conservative substitutions relative to (i);
   X₂₅ is selected from the group consisting of (i) amino acid residues E, Q, D and (ii) amino acid residues that are conservative substitutions relative to (i);
   X₂₆ is selected from the group consisting of (i) amino acid residue A, S and (ii) amino acid residues that are conservative substitutions relative to (i);
   X₂₇ is selected from the group consisting of (i) amino acid residues R, H, V and (ii) amino acid residues that are conservative substitutions relative to (i);
   X₂₈ is selected from the group consisting of (i) amino acid residues S, L, A and (ii) amino acid residues that are conservative substitutions relative to (i);
   X₂₉ is selected from the group consisting of (i) amino acid residue F, Y and (ii) amino acid residues that are conservative substitutions relative to (i).

### (ADI-47754 family)

In certain embodiments, the antibody or antigen-binding fragment thereof comprises:
(1) the following three complementary determining regions (CDR) of a heavy chain variable region (VH):
   (a) a VH CDR1, which has a structure as shown in FTFX₁X₂YX₃MH (SEQ ID NO: 76);
   (b) a VH CDR2, which has a structure as as shown in VIX₄YX₅X₆X₇X₈KYYAX₉SVKG (SEQ ID NO: 77);
   (c) a VH CDR3, which has a structure as shown in ARDTSRGHDI (SEQ ID NO: 23); and/or,
(2) the following three complementary determining regions (CDR) of a light chain variable region (VL):
   (d) a VL CDR1, which has a structure as shown in X₁₂ASQSVSX₁₃X₁₄YLA (SEQ ID NO: 79);
   (e) a VL CDR2, which has a structure as shown in GASX₁₈X₁₉X₂₀X₂₁ (SEQ ID NO: 8);
   (f) a VL CDR3, which has a structure as shown in X₂₄X₂₅YX₂₆DYPPFT (SEQ ID NO: 10);
   wherein,
   X₁ is selected from the group consisting of (i) amino acid residues S, E, G and (ii) amino acid residues that are conservative substitutions relative to (i);
   X₂ is selected from the group consisting of (i) amino acid residues S, K and (ii) amino acid residues that are conservative substitutions relative to (i);
   X₃ is selected from the group consisting of (i) amino acid residues G and (ii) amino acid residues that are conservative substitutions relative to (i);
   X₄ is selected from the group consisting of (i) amino acid residues S, H and (ii) amino acid residues that are conservative substitutions relative to (i);
   X₅ is selected from the group consisting of (i) amino acid residues E, H and (ii) amino acid residues that are conservative substitutions relative to (i);
   X₆ is selected from the group consisting of (i) amino acid residue G, A and (ii) amino acid residues that are conservative substitutions relative to (i);
   X₇ is selected from the group consisting of (i) amino acid residues S, E, T, V and (ii) amino acid residues that are conservative substitutions relative to (i);
   X₈ is selected from the group consisting of (i) amino acid residues N, S, I, K and (ii) amino acid residues that are conservative substitutions relative to (i);
   X₉ is selected from the group consisting of (i) amino acid residues D, E and (ii) amino acid residues that are conservative substitutions relative to (i);
   X₁₂ is selected from the group consisting of (i) amino acid residues R, Q and (ii) amino acid residues that are conservative substitutions relative to (i);
   X₁₃ is selected from the group consisting of (i) amino acid residues Y, S and (ii) amino acid residues that are conservative substitutions relative to (i);
   X₁₄ is selected from the group consisting of (i) amino acid residues S, D, N and (ii) amino acid residues that are conservative substitutions relative to (i);
   X₁₈ is selected from the group consisting of (i) amino acid residues S, T, A and (ii) amino acid residues that are conservative substitutions relative to (i);
   X₁₉ is selected from the group consisting of (i) amino acid residues R, L and (ii) amino acid residues that are conservative substitutions relative to (i);
   X₂₀ is selected from the group consisting of (i) amino acid residues N, A and (ii) amino acid residues that are conservative substitutions relative to (i);
   X₂₁ is selected from the group consisting of (i) amino acid residues T, N and (ii) amino acid residues that are conservative substitutions relative to (i);
   X₂₄ is selected from the group consisting of (i) amino acid residues S, G, Q and (ii) amino acid residues that are conservative substitutions relative to (i);
   X₂₅ is selected from the group consisting of (i) amino acid residues E, Q, D and (ii) amino acid residues that are conservative substitutions relative to (i);
   X₂₆ is selected from the group consisting of (i) amino acid residue A, S and (ii) amino acid residues that are conservative substitutions relative to (i).

In certain embodiments, X₁ is selected from the group consisting of amino acid residues S, E, G; X₂ is selected from the group consisting of amino acid residues S, K; X₃ is amino acid residue G; X₄ is selected from the group consisting of amino acid residues S, H; X₅ is selected from the group consisting of amino acid residues E, H; X₆ is selected from the group consisting of amino acid residues G, A; X₇ is selected from the group consisting of amino acid residues S, E, T, V; X₈ is selected from the group consisting of amino acid residues N, S, I, K; X₉ is selected from the group consisting of amino acid residues D, E; X₁₂ is selected from the group consisting of amino acid residues R, Q; X₁₃ is selected from the group consisting of amino acid residues Y, S; X₁₄ is selected from the group consisting of amino acid residues S, D, N; X₁₈ is selected from the group consisting of amino acid residues S, T, A; X₁₉ is selected from the group consisting of amino acid residues R, L; X₂₀ is selected from the group consisting of amino acid residues N, A; X₂₁ is selected from the group consisting of amino acid residues T, N; X₂₄ is selected from the group consisting of amino acid residues S, G, Q; X₂₅ is selected from the group consisting of amino acid residues E, Q, D; X₂₆ is selected from the group consisting of amino acid residues A, S.

In certain embodiments, the antibody or antigen-binding fragment thereof comprises:
a VH CDR1 as shown in any one of SEQ ID NOs: 1-3; a VH CDR2 as shown in any one of SEQ ID NOs: 12-18, 82; and a VH CDR3 as shown in SEQ ID NO: 23; a VL CDR1 as shown in any one of SEQ ID NOs: 37-40; a VL CDR2 as shown in any one of SEQ ID NOs: 45-50; and a VL CDR3 as shown in any one of SEQ ID NOs: 54-57;
In certain embodiments, the antibody or antigen-binding fragment thereof comprises:
   (1) a VH CDR1 as shown in SEQ ID NO: 1; a VH CDR2 as shown in SEQ ID NO: 12; and, a VH CDR3 as shown in SEQ ID NO: 23; a VL CDR1 as shown in SEQ ID NO: 37; a VL CDR2 as shown in SEQ ID NO: 45; and, a VL CDR3 as shown in SEQ ID NO: 54;
   (2) a VH CDR1 as shown in SEQ ID NO: 1; a VH CDR2 as shown in SEQ ID NO: 13; and, a VH CDR3 as shown in SEQ ID NO: 23; a VL CDR1 as shown in SEQ ID NO: 37; a VL CDR2 as shown in SEQ ID NO: 45; and, a VL CDR3 as shown in SEQ ID NO: 54;
   (3) a VH CDR1 as shown in SEQ ID NO: 2; a VH CDR2 as shown in SEQ ID NO: 14; and, a VH CDR3 as shown in SEQ ID NO: 23; a VL CDR1 as shown in SEQ ID NO: 38; a VL CDR2 as shown in SEQ ID NO: 46; and, a VL CDR3 as shown in SEQ ID NO: 55;
   (4) a VH CDR1 as shown in SEQ ID NO: 2; a VH CDR2 as shown in SEQ ID NO: 15; and, a VH CDR3 as shown in SEQ ID NO: 23; a VL CDR1 as shown in SEQ ID NO: 39; a VL CDR2 as shown in SEQ ID NO: 47; and, a VL CDR3 as shown in SEQ ID NO: 56;
   (5) a VH CDR1 as shown in SEQ ID NO: 2; a VH CDR2 as shown in SEQ ID NO: 16; and, a VH CDR3 as shown in SEQ ID NO: 23; a VL CDR1 as shown in SEQ ID NO: 39; a VL CDR2 as shown in SEQ ID NO: 48; and, a VL CDR3 as shown in SEQ ID NO: 57;
   (6) a VH CDR1 as shown in SEQ ID NO: 2; a VH CDR2 as shown in SEQ ID NO: 17; and, a VH CDR3 as shown in SEQ ID NO: 23; a VL CDR1 as shown in SEQ ID NO: 40; a VL CDR2 as shown in SEQ ID NO: 49; and, a VL CDR3 as shown in SEQ ID NO: 57;
   (7) a VH CDR1 as shown in SEQ ID NO: 3; a VH CDR2 as shown in SEQ ID NO: 18; and, a VH CDR3 as shown in SEQ ID NO: 23; a VL CDR1 as shown in SEQ ID NO: 38; a VL CDR2 as shown in SEQ ID NO: 50; and, a VL CDR3 as shown in SEQ ID NO: 54; or
   (8) a VH CDR1 as shown in SEQ ID NO: 2; a VH CDR2 as shown in SEQ ID NO: 82; and, a VH CDR3 as shown in SEQ ID NO: 23; a VL CDR1 as shown in SEQ ID NO: 39; a VL CDR2 as shown in SEQ ID NO: 48; and, a VL CDR3 as shown in SEQ ID NO: 57.

In certain embodiments, the antibody or antigen-binding fragment thereof further comprises a framework region of a human immunoglobulin. For example, the antibody or antigen-binding fragment thereof comprises a framework region contained in an amino acid sequence encoded by a human germline antibody gene; for example, the antibody or antigen-binding fragment thereof comprises: a heavy chain framework region comprised in an amino acid sequence encoded by a human heavy chain germline gene, and/or a light chain framework region comprised in an amino acid sequence encoded by a human light chain germline gene.

In certain embodiments, the antibody or antigen-binding fragment thereof comprises:
(1) a heavy chain variable region (VH) comprising an amino acid sequence selected from the following:
   (i) the sequence as shown in any one of SEQ ID NOs:26-32, 83;
   (ii) a sequence having a substitution, deletion, or addition of one or several amino acids (e.g., a substitution, deletion or addition of 1, 2, 3, 4 or 5 amino acids) as compared to the sequence shown in any one of SEQ ID NOs: 26-32, 83; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 26-32, 83;
      and/or,
(2) a light chain variable region (VL) comprising an amino acid sequence selected from the following:
   (i) the sequence as shown in any one of SEQ ID NOs:61-66;
   (ii) a sequence having a substitution, deletion, or addition of one or several amino acids (e.g., a substitution, deletion or addition of 1, 2, 3, 4 or 5 amino acids) as compared to the sequence shown in any one of SEQ ID NOs: 61-66; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 61-66.

In certain embodiments, the antibody or antigen-binding fragment thereof comprises a VH comprising the sequence as shown in any one of SEQ ID NOs: 26-32, 83 or variant thereof, and a VL comprising the sequence as shown in any one of SEQ ID NOs: 61-66 or variant thereof;
wherein, the variant has a substitution, deletion, or addition of one or several amino acids (e.g., a substitution, deletion or addition of 1, 2, 3, 4 or 5 amino acids), or has a sequence identity of at at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived. In certain embodiments, the substitution is a conservative substitution.

In certain embodiments, the antibody or antigen-binding fragment thereof comprises a VH of the sequence shown in SEQ ID NO: 26 or variant thereof, and a VL of the sequence shown in SEQ ID NO: 61 or variant thereof.

In certain embodiments, the antibody or antigen-binding fragment thereof comprises a VH of the sequence shown in SEQ ID NO: 27 or variant thereof, and a VL of the sequence shown in SEQ ID NO: 61 or variant thereof.

In certain embodiments, the antibody or antigen-binding fragment thereof comprises a VH of the sequence shown in SEQ ID NO: 28 or variant thereof, and a VL of the sequence shown in SEQ ID NO: 62 or variant thereof.

In certain embodiments, the antibody or antigen-binding fragment thereof comprises a VH of the sequence shown in SEQ ID NO: 29 or variant thereof, and a VL of the sequence shown in SEQ ID NO: 63 or variant thereof.

In certain embodiments, the antibody or antigen-binding fragment thereof comprises a VH of the sequence shown in SEQ ID NO: 30 or a variant thereof, and a VL of the sequence shown in SEQ ID NO: 64 or a variant thereof.

In certain embodiments, the antibody or antigen-binding fragment thereof comprises a VH of the sequence shown in SEQ ID NO: 31 or variant thereof, and a VL of the sequence shown in SEQ ID NO:65 or variant thereof.

In certain embodiments, the antibody or antigen-binding fragment thereof comprises a VH of the sequence shown in SEQ ID NO: 32 or variant thereof, and a VL of the sequence shown in SEQ ID NO: 66 or variant thereof.

In certain embodiments, the antibody or antigen-binding fragment thereof comprises a VH of the sequence shown in SEQ ID NO: 83 or variant thereof, and a VL of the sequence shown in SEQ ID NO: 64 or variant thereof;
wherein, the variant has a substitution, deletion, or addition of one or several amino acids (e.g., a substitution, deletion or addition of 1, 2, 3, 4 or 5 amino acids), or has a sequence identity of at at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived. In certain embodiments, the substitution is a conservative substitution.

In certain embodiments, the antibody or antigen-binding fragment thereof is capable of blocking the binding of CD40 to its ligand CD40L.

### (ADI-47720 family)

In some embodiments, the antibody or antigen-binding fragment thereof comprises:
(1) the following three complementarity-determining regions (CDR) of a heavy chain variable region (VH):
   (a) a VH CDR1, which has a structure as shown in FTFX₁X₂YX₃MH (SEQ ID NO: 76);
   (b) a VH CDR2, which has a structure as shown in GISWSSX₁₀SIX₁₁YADSVKG (SEQ ID NO: 78);
   (c) a VH CDR3, which has a structure as shown in AKDPTATTGARGYFDL (SEQ ID NO: 24);
      and/or,
(2) the following three complementarity-determining regions (CDR) of a light chain variable region (VL):
   (d) a VL CDR1, which has a structure as shown in X₁₅ASX₁₆X₁₇ISSWLA (SEQ ID NO: 7);
   (e) a VL CDR2, which has a structure as shown in AASX₂₂LX₂₃S (SEQ ID NO: 9);
   (f) a VL CDR3, which has a structure as shown in QQX₂₇X₂₈SX₂₉PPT (SEQ ID NO: 11);
   wherein,
   X₁ is selected from the group consisting of (i) amino acid residues D and (ii) amino acid residues that are conservative substitutions relative to (i);
   X₂ is selected from the group consisting of (i) amino acid residues D, T and (ii) amino acid residues that are conservative substitutions relative to (i);
   X₃ is selected from the group consisting of (i) amino acid residues A and (ii) amino acid residues that are conservative substitutions relative to (i);
   X₁₀ is selected from the group consisting of (i) amino acid residues G, D and (ii) amino acid residues that are conservative substitutions relative to (i);
   X₁₁ is selected from the group consisting of (i) amino acid residues G, H and (ii) amino acid residues that are conservative substitutions relative to (i);
   X₁₅ is selected from the group consisting of (i) amino acid residues R, E and (ii) amino acid residues that are conservative substitutions relative to (i);
   X₁₆ is selected from the group consisting of (i) amino acid residues Q, K and (ii) amino acid residues that are conservative substitutions relative to (i);
   X₁₇ is selected from the group consisting of (i) amino acid residues G, V and (ii) amino acid residues that are conservative substitutions relative to (i);
   X₂₂ is selected from the group consisting of (i) amino acid residue S. Y and (ii) amino acid residues that are conservative substitutions relative to (i);
   X₂₃ is selected from the group consisting of (i) amino acid residue E, Q and (ii) amino acid residues that are conservative substitutions relative to (i);
   X₂₇ is selected from the group consisting of (i) amino acid residues R, H and (ii) amino acid residues that are conservative substitutions relative to (i);
   X₂₈ is selected from the group consisting of (i) amino acid residues S, A and (ii) amino acid residues that are conservative substitutions relative to (i);
   X₂₉ is selected from the group consisting of (i) amino acid residues F and (ii) amino acid residues that are conservative substitutions relative to (i).

In certain embodiments, X₁ is amino acid residue D; X₂ is selected from the group consisting of amino acid residues D, T; X₃ is amino acid residue A; X₁₀ is selected from the group consisting of amino acid residues G, D; X₁₁ is selected from the group consisting of amino acid residues G, H; X₁₅ is selected from the group consisting of amino acid residues R, E; X₁₆ is selected from the group consisting of amino acid residues Q, K; X₁₇ is selected from the group consisting of amino acid residues G, V; X₂₂ is selected from the group consisting of amino acid residues S, Y; X₂₃ is selected from the group consisting of amino acid residues E, Q; X₂₇ is selected from the group consisting of amino acid residues R, H; X₂₈ is selected from the group consisting of amino acid residues S, A; X₂₉ is amino acid residue F.

In certain embodiments, the antibody or antigen-binding fragment thereof comprises a VH CDR1 as shown in SEQ ID NOs: 4 or 5; a VH CDR2 as shown in any one of SEQ ID NOs: 19-21; and, a VH CDR3 as shown in SEQ ID NO: 24; a VL CDR1 as shown in any one of SEQ ID NOs: 41-43; a VL CDR2 as shown in any one of SEQ ID NO: 51-53; and, a VL CDR3 as shown in SEQ ID NO: 58 or 59.

In certain embodiments, the antibody or antigen-binding fragment thereof comprises:
(1) a VH CDR1 as shown in SEQ ID NO: 4; a VH CDR2 as shown in SEQ ID NO: 19; and a VH CDR3 as shown in SEQ ID NO: 24; a VL CDR1 as shown in SEQ ID NO: 41; a VL CDR2 as shown in SEQ ID NO: 51; and a VL CDR3 as shown in SEQ ID NO: 58;
(2) a VH CDR1 as shown in SEQ ID NO: 4; a VH CDR2 as shown in SEQ ID NO: 20; and a VH CDR3 as shown in SEQ ID NO: 24; a VL CDR1 as shown in SEQ ID NO: 42; a VL CDR2 as shown in SEQ ID NO: 52; and a VL CDR3 as shown in SEQ ID NO: 59;
   or,
(3) a VH CDR1 as shown in SEQ ID NO: 5; a VH CDR2 as shown in SEQ ID NO: 21; and a VH CDR3 as shown in SEQ ID NO: 24; a VL CDR1 as shown in SEQ ID NO: 43; a VL CDR2 as shown in SEQ ID NO: 53; and a VL CDR3 as shown in SEQ ID NO: 59.

In certain embodiments, the antibody or antigen-binding fragment thereof further comprises a framework region of a human immunoglobulin. For example, the antibody or antigen-binding fragment thereof comprises a framework region contained in an amino acid sequence encoded by a human germline antibody gene; for example, the antibody or antigen-binding fragment thereof comprises: a heavy chain framework region comprised in an amino acid sequence encoded by a human heavy chain germline gene, and/or a light chain framework region comprised in an amino acid sequence encoded by a human light chain germline gene.

In certain embodiments, the antibody or antigen-binding fragment thereof comprises:
(1) a heavy chain variable region (VH) comprising an amino acid sequence selected from the following:
   (i) a sequence as shown in any one of SEQ ID NOs: 33-35;
   (ii) a sequence having a substitution, deletion, or addition of one or several amino acids (e.g., a substitution, deletion or addition of 1, 2, 3, 4 or 5 amino acids) as compared to the sequence shown in any one of SEQ ID NOs: 33-35; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 33-35;
      and/or,
(2) a light chain variable region (VL) comprising an amino acid sequence selected from the following:
   (i) a sequence as shown in any one of SEQ ID NOs: 67-69;
   (ii) a sequence having a substitution, deletion, or addition of one or several amino acids (e.g., a substitution, deletion or addition of 1, 2, 3, 4 or 5 amino acids) as compared to the sequence shown in any one SEQ ID NOs: 67-69; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 67-69.

In certain embodiments, the antibody or antigen-binding fragment thereof comprises a VH of the sequence as shown in any one of SEQ ID NOs: 33-35 or variant thereof, and a VL of the sequence as shown in any one of SEQ ID NOs: 67-69 or variant thereof;
wherein, the variant has a substitution, deletion, or addition of one or several amino acids (e.g., a substitution, deletion or addition of 1, 2, 3, 4 or 5 amino acids), or has a sequence identity of at at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived. In certain embodiments, the substitution is a conservative substitution.

In certain embodiments, the antibody or antigen-binding fragment thereof comprises a VH of the sequence as shown in SEQ ID NO: 33 or variant thereof, and a VL of the sequence as shown in SEQ ID NO: 67 or variant thereof.

In certain embodiments, the antibody or antigen-binding fragment thereof comprises a VH of the sequence as shown in SEQ ID NO: 34 or variant thereof, and a VL of the sequence as shown in SEQ ID NO: 68 or variant thereof.

In certain embodiments, the antibody or antigen-binding fragment thereof comprises a VH of the sequence as shown in SEQ ID NO: 35 or variant thereof, and, a VL of the sequence as shown in SEQ ID NO: 69 or variant thereof.
wherein, the variant has a substitution, deletion, or addition of one or several amino acids (e.g., a substitution, deletion or addition of 1, 2, 3, 4 or 5 amino acids), or has a sequence identity of at at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived. In certain embodiments, the substitution is a conservative substitution.

In some embodiments, the antibody or antigen-binding fragment thereof non-blocks the binding of human CD40 to its ligand CD40L.

### (ADI-47741 family)

In certain embodiments, the antibody or antigen-binding fragment thereof comprises:
(1) the following three complementarity-determining regions (CDR) of a heavy chain variable region (VH):
   (a) a VH CDR1, which has a structure as shown in GSISSYYWS (SEQ ID NO: 6);
   (b) a VH CDR2, which has a structure as shown in SIYYSGSTNYNPSLKS (SEQ ID NO: 22);
   (c) a VH CDR3, which has a structure as shown in ARDANYYKWHPY (SEQ ID NO: 25); and/or,
(2) the following three complementarity-determining regions (CDR) of a light chain variable region (VL):
   (d) a VL CDR1, which has a structure as shown in X₁₂ASQSVSX₁₃X₁₄YLA (SEQ ID NO: 79);
   (e)a VL CDR2, which has a structure as shown in GASX₁₈X₁₉X₂₀X₂₁ (SEQ ID NO: 8);
   (f) a VL CDR3, which has a structure as shown in QQX₂₇X₂₈SX₂₉PPT (SEQ ID NO: 11);
   wherein,
   X₁₂ is selected from the group consisting of (i) amino acid residue R and (ii) amino acid residues that are conservative substitutions relative to (i);
   X₁₃ is selected from the group consisting of (i) amino acid residue S and (ii) amino acid residues that are conservative substitutions relative to (i);
   X₁₄ is selected from the group consisting of (i) amino acid residue D and (ii) amino acid residues that are conservative substitutions relative to (i);
   X₁₈ is selected from the group consisting of (i) amino acid residue S and (ii) amino acid residues that are conservative substitutions relative to (i);
   X₁₉ is selected from the group consisting of (i) amino acid residue R and (ii) amino acid residues that are conservative substitutions relative to (i);
   X₂₀ is selected from the group consisting of (i) amino acid residue A and (ii) amino acid residues that are conservative substitutions relative to (i);
   X₂₁ is selected from the group consisting of (i) amino acid residue T and (ii) amino acid residues that are conservative substitutions relative to (i);
   X₂₇ is selected from the group consisting of (i) amino acid residue V and (ii) amino acid residues that are conservative substitutions relative to (i);
   X₂₈ is selected from the group consisting of (i) amino acid residue L and (ii) amino acid residues that are conservative substitutions relative to (i);
   X₂₉ is selected from the group consisting of (i) amino acid residue Y and (ii) amino acid residues that are conservative substitutions relative to (i).

In certain embodiments, X₁₂ is amino acid residue R; X₁₃ is amino acid residue S; X₁₄ is amino acid residue D; X₁₈ is amino acid residue S; X₁₉ is amino acid residue R; X₂₀ is amino acid residue A; X₂₁ is amino acid residue T; X₂₇ is amino acid residue V; X₂₈ is amino acid residue L; and X₂₉ is amino acid residue Y.

In certain embodiments, the antibody or antigen-binding fragment thereof comprises a VH CDR1 as shown in SEQ ID NO: 6; a VH CDR2 as shown in SEQ ID NO: 22; and, a VH CDR3 as shown in SEQ ID NO: 25; a VL CDR1 as shown in SEQ ID NO: 44; a VL CDR2 as shown in SEQ ID NO: 45; and a VL CDR3 as shown in SEQ ID NO: 60.

In certain embodiments, the antibody or antigen-binding fragment thereof further comprises a framework region of a human immunoglobulin. For example, the antibody or antigen-binding fragment thereof comprises a framework region contained in an amino acid sequence encoded by a human germline antibody gene; for example, the antibody or antigen-binding fragment thereof comprises: a heavy chain framework region comprised in an amino acid sequence encoded by a human heavy chain germline gene, and/or a light chain framework region comprised in an amino acid sequence encoded by a human light chain germline gene.

In certain embodiments, the antibody or antigen-binding fragment thereof comprises:
(1) a heavy chain variable region (VH) comprising an amino acid sequence selected from the following:
   (i) a sequence as shown in SEQ ID NO: 36;
   (ii) a sequence having a substitution, deletion, or addition of one or several amino acids (e.g., a substitution, deletion or addition of 1, 2, 3, 4 or 5 amino acids) as compared to the sequence shown in SEQ ID NO: 36; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 36;
      and/or,
(2) a light chain variable region (VL) comprising an amino acid sequence selected from the following:
   (i) a sequence as shown in SEQ ID NO: 70;
   (ii) a sequence having a substitution, deletion, or addition of one or several amino acids (e.g., a substitution, deletion or addition of 1, 2, 3, 4 or 5 amino acids) as compared to the sequence shown in SEQ ID NO: 70; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 70.

In certain embodiments, the antibody or antigen-binding fragment thereof comprises:
a VH of the sequence as shown in SEQ ID NO: 36 or variant thereof, and a VL of the sequence as shown in SEQ ID NO: 70 or variant thereof;
wherein, the variant has a substitution, deletion, or addition of one or several amino acids (e.g., a substitution, deletion or addition of 1, 2, 3, 4 or 5 amino acids), or has a sequence identity of at at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived. In certain embodiments, the substitution is a conservative substitution.

In certain embodiments, the antibody or antigen-binding fragment thereof is capable of blocking the binding of human CD40 to its ligand CD40L.

In certain embodiments, the antibody or antigen-binding fragment thereof further comprises a constant region derived from a human immunoglobulin.

In certain embodiments, the heavy chain of the antibody or antigen-binding fragment thereof comprises the heavy chain constant region (CH) of a human immunoglobulin or variant thereof, the variant has a substitution, deletion or addition of one or several amino acids (e.g., a substitution, deletion or addition of up to 20, up to 15, up to 10, or up to 5 amino acids; for example, a substitution, deletion or addition of 1, 2, 3, 4 or 5 amino acids) as compared to the sequence from which it is derived; and/or,
the light chain of the antibody or antigen-binding fragment thereof comprises the light chain constant region (CL) of a human immunoglobulin or variant thereof, and the variant has a conservative substitution of up to 20 amino acids (e.g., a conservative substitution of up to 15, up to 10, or up to 5 amino acids; such as a conservative substitution of 1, 2, 3, 4 or 5 amino acids) as compared to the sequence from which it is derived.

In some embodiments, the variant of the heavy chain constant region (CH) may have a conservative substitution of one or more amino acids as compared to the sequence from which it is derived. In such embodiments, the variant of the heavy chain constant region (CH) may have the same or substantially the same effector function as compared to the wild-type sequence from which it is derived.

In other embodiments, the variant of the heavy chain constant region (CH) may comprise one or more amino acid mutations or chemical modifications to alter one or more of the following properties of the antibody of the present invention: Fc receptor binding, antibody glycosylation, number of cysteine residues, effector cell function, or complement function, etc. A functional change can be achieved by replacing at least one amino acid residue in the constant region of the antibody with a different residue or by chemically modifying at least one amino acid residue of the antibody, for example, changing the affinity of the antibody to an effector ligand (e.g., FcR or complement Cl q), thereby altering (e.g., reducing or enhancing) effector function, such as several important effector functions mediated by the Fc region of the antibody, including ADCC, phagocytosis, CDC, etc.

In certain embodiments, the antibody or antigen-binding fragment thereof possesses ADCC activity. In certain embodiments, the antibody or antigen-binding fragment thereof comprises a mutated (e.g., amino acid substitution) or chemically modified Fc region, wherein the mutated or chemically modified Fc region provides enhanced ADCC activity. In certain embodiments, the antibody or antigen-binding fragment thereof is produced by expression in a host cell with low or no fucosylation activity, and the antibody produced has low or no fucosylation, which enhances its ADCC activity. Such host cells may be a mammalian cell, such as CHO cell, which is lack of a gene encoding a fucosyltransferase (e.g., FUT8).

In certain embodiments, the heavy chain of the antibody or antigen-binding fragment thereof comprises a heavy chain constant region derived from a human immunoglobulin (e.g., IgG1, IgG2, IgG3, or IgG4).

In certain exemplary embodiments, the heavy chain of the antibody or antigen-binding fragment thereof comprises a heavy chain constant region as shown in SEQ ID NO: 155.

In certain embodiments, the light chain of the antibody or antigen-binding fragment thereof comprises a light chain constant region (e.g., a constant region of κ or λ chain) derived from a human immunoglobulin.

In certain exemplary embodiments, the light chain of the antibody or antigen-binding fragment thereof comprises a light chain constant region as shown in SEQ ID NO: 156.

In certain embodiments, the antibody or antigen-binding fragment thereof is selected from the group consisting of Fab, Fab', F(ab')₂ , Fv, disulfide-linked Fv, scFv, diabody, and single-domain antibody (sdAb); and/or, the antibody is a chimeric antibody, a bispecific antibody or multi-specific antibody.

In certain embodiments, the antibody or antigen-binding fragment thereof possesses cross-reactivity with humanand cynomolgus CD40.

The antibody of the present invention can be prepared by various methods known in the art, such as by genetic engineering and recombinant technology. For example, DNA molecules encoding the heavy chain and light chain of the antibody of the present invention are obtained by chemical synthesis or PCR amplification. The resulting DNA molecule is inserted into an expression vector and then transfected into host cells. Then, the transfected host cells are cultured under certain conditions to express the antibody of the present invention.

The antigen-binding fragments of the present invention can be obtained by hydrolyzing intact antibody molecules (see Morimoto et al., J. Biochem. Biophys. Methods 24:107-117 (1992) and Brennan et al., Science 229:81 (1985)). Besides, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from the cell culture of the recombinant host cell. Those of ordinary skill in the art are well aware of other techniques for preparing such antigen-binding fragments.

### Isolated Nucleic Acid Molecules

In another aspect, the present application also provides an isolated nucleic acid molecule, which encodes the antibody or antigen-binding fragment thereof as described above, or heavy chain variable region and/or light chain variable region thereof.

In certain embodiments, the isolated nucleic acid molecule comprises a first nucleotide sequence encoding a heavy chain or heavy chain variable region of the antibody or antigen-binding fragment thereof of the present invention and a second nucleotide sequence encoding a light chain or light chain variable region of the antibody or antigen-binding fragment thereof, wherein the first nucleotide sequence and the second nucleotide sequence are present on the same or different isolated nucleic acid molecules. When the first nucleotide sequence and the second nucleotide sequence are present on different isolated nucleic acid molecules, the isolated nucleic acid molecule of the present invention includes a first nucleic acid molecule comprising the first nucleotide sequence and a second nucleic acid molecule comprising the second nucleotide sequence.

### Vectors

In another aspect, the present application provides a vector, which comprises the nucleic acid molecule as described above. In certain embodiments, the vector is a cloning vector or an expression vector.

In certain embodiments, the vector comprises a first nucleotide sequence encoding a heavy chain or heavy chain variable region of the antibody or antigen-binding fragment thereof of the present invention and a second nucleotide sequence encoding a light chain or light chain variable region of the antibody or antigen-binding fragment thereof, wherein the first nucleotide sequence and the second nucleotide sequence are present on the same or different vectors. When the first nucleotide sequence and the second nucleotide sequence are present on different vectors, the vector of the present invention comprises a first vector comprising the first nucleotide sequence and a second vector comprising the second nucleotide sequence.

### Host Cells

In another aspect, the present application also provides a host cell, which comprises the nucleic acid molecule or vector as described above. Such host cell includes, but is not limited to, prokaryotic cell such as bacterial cell (e.g., *E. coli* cell), and eukaryotic cell such as fungal cell (e.g., yeast cell), insect cell, plant cell, and animal cell (e.g., mammalian cell, for example, mouse cell, human cell, etc.).

### Preparation Method

In another aspect, the present application provides a method for preparing the antibody or antigen-binding fragment thereof as described above, which comprises culturing the host cell as described above under a condition that allows expression of the antibody or antigen-binding fragment thereof, and recovering the antibody or antigen-binding fragment thereof from a cell culture of the host cell.

### Therapeutic uses

In another aspect, the present application provides a pharmaceutical composition, which comprises the antibody or antigen-binding fragment thereof as described above, and a pharmaceutically acceptable carrier and/or excipient.

In certain exemplary embodiments, the pharmaceutically acceptable carrier and/or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

In another aspect, the present application provides a use of the antibody or antigen-binding fragment thereof, the isolated nucleic acid molecule, the vector, the host cell or the pharmaceutical composition as described above in the manufacture of a medicament, and the medicament is used for activating CD40, increasing an immune cell activity, enhancing an immune response, and/or preventing and/or treating a tumor or an infection in a subject.

In certain embodiments, the immune cells is a T cell, a B cell, a DC, a macrophage and/or a NK cell.

In certain embodiments, the immune response is a CD40-mediated immune response.

In certain embodiments, the tumor is selected from a solid tumor or a hematological tumor (e.g., leukemia, lymphoma, myeloma). In certain embodiments, the tumor is selected from a solid tumor or a lymphoma.

In certain embodiments, the tumor is selected from the group consisting of colorectal cancer, colon cancer, bladder cancer, breast cancer, uterine/cervical cancer, ovarian cancer, prostate cancer, testicular cancer, esophageal cancer, gastrointestinal cancer, pancreatic cancer, renal cancer, head and neck cancer, lung cancer, stomach cancer, germ cell cancer, bone cancer, liver cancer, thyroid cancer, skin cancer, central nervous system cancer, lymphoma, leukemia, myeloma, sarcoma, and melanoma.

In certain embodiments, the infection is selected from the group consisting of viral infection, bacterial infection, fungal infection, and parasitic infection.

In certain embodiments, the subject is a mammal, such as a human.

In some embodiments, the antibody or antigen-binding fragment thereof is used alone or in combination with an additional pharmaceutically active agent.

In another aspect, the present application provides a method for enhancing an immune response and/or preventing and/or treating a tumor or infection in a subject, which comprises: administering to the subject in need thereof an effective amount of the antibody or antigen-binding fragment thereof or the pharmaceutical compositions as described above.

In some embodiments, the immune response is a CD40-mediated immune response.

In certain embodiments, the tumor is selected from a solid tumor or a hematological tumor (e.g., leukemia, lymphoma, myeloma). In certain embodiments, the tumor is selected from a solid tumor or a lymphoma.

In certain embodiments, the tumor is selected from the group consisting of colorectal cancer, colon cancer, bladder cancer, breast cancer, uterine/cervical cancer, ovarian cancer, prostate cancer, testicular cancer, esophageal cancer, gastrointestinal cancer, pancreatic cancer, renal cancer, head and neck cancer, lung cancer, gastric cancer, germ cell carcinoma, bone cancer, liver cancer, thyroid carcinoma, skin cancer, tumors of the central nervous system, lymphomas, leukemias, myelomas, sarcomas, and melanomas.

In certain embodiments, the infection is selected from the group consisting of viral infection, bacterial infection, fungal infection, and parasitic infection.

In certain embodiments, said subject is a mammal, such as a human.

The antibody or antigen-binding fragment thereof or the pharmaceutical composition of the present application can be formulated into any dosage form known in the medical field, for example, tablet, pill, suspension, emulsion, solution, gel, capsule, powder, granule, elixir, lozenge, suppository, injection (including injectable solution, sterile powder for injection and concentrated solution for injection), inhalant, spray, etc. The preferred dosage form depends on the intended mode of administration and therapeutic use. The antibody or antigen-binding fragment thereof or pharmaceutical composition of the present invention should be sterile and stable under the conditions of production and storage. One preferred dosage form is an injection. Such injection may be a sterile injectable solution. For example, the sterile injectable solution may be prepared by incorporating in an appropriate solvent the requisite dose of the antibody or antigen-binding fragment thereof of the present invention, and, optionally, other desired ingredients (including, but not limited to, pH adjuster, surfactant, adjuvant, ionic strength enhancer, isotonic agent, preservative, diluent, or any combination thereof), followed by filter sterilization. Additionally, the sterile injectable solution may be prepared as a sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution and any combination thereof.

The antibody or antigen-binding fragment thereof of the present application, or the pharmaceutical compositions of the present invention may be administered by any suitable method known in the art, including but not limited to, oral, buccal, sublingual, ophthalmic, local, parenteral, rectal, intrathecal, intra-cisternal, inguinal, intravesical, topical (e.g., powder, ointment, or drop), or nasal route. However, for many therapeutic uses, the preferred route/mode of administration is parenteral administration (e.g., intravenous or bolus injection, subcutaneous injection, intraperitoneal injection, intramuscular injection). Those skilled in the art will understand that the route and/or mode of administration will vary depending on the intended purpose. In certain embodiments, the antibody or antigen-binding fragment thereof or the pharmaceutical composition of the present invention is administered by intravenous injection or bolus injection.

### Detection uses

In another aspect, the present application provides a conjugate, which comprises the antibody or antigen-binding fragment thereof as described above, and a detectable label linked to the antibody or antigen-binding fragment thereof.

In certain embodiments, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide and biotin.

In another aspect, the present application provides a kit, which comprises the antibody or antigen-binding fragment thereof or the conjugate as described above.

In certain embodiments, the kit comprises the conjugate as described above.

In certain embodiments, the kit comprises the antibody or antigen-binding fragment thereof as described above, and a second antibody that specifically recognizes the antibody or antigenbinding fragment thereof. In certain embodiments, the second antibody further comprises a detectable label, such as an enzyme (e.g., horseradish peroxidase or alkaline phosphatase), a chemiluminescent reagent (e.g., acridinium esters, luminol and derivative thereof, or ruthenium derivative), fluorescent dye (e.g., fluorescein or fluorescent protein), radionuclide or biotin.

In another aspect, the present application provides a method for detecting the presence or level of CD40 in a sample, which comprises using the antibody or antigen-binding fragment thereof as described above or the conjugate as described above. In certain embodiments, the method is used for a therapeutic purpose, a diagnostic purposes, or a non-therapeutic non-diagnostic purpose.

In certain embodiments, the method is an immunological assay, such as a Western blot, an enzyme immunoassay (e.g., ELISA), a chemiluminescent immunoassay, a fluorescent immunoassay, or a radioimmunoassay.

In certain embodiments, the method comprises using the conjugate as described above.

In certain embodiments, the method comprising using the antibody or antigen-binding fragment thereof as described above, and the method further comprises using a second antibody carrying a detectable label (e.g., an enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide, or biotin) to detect the antibody or antigen-binding fragment thereof.

In certain embodiments, the method comprises: (1) contacting the sample with the antibody or antigen-binding fragment thereof or the conjugate of the present invention; (2) detecting the formation of an antigen-antibody immune complex or detecting an amount of the immune complex. The formation of the immune complex indicates the presence of CD40 or CD40-expressing cells.

In another aspect, the present application also provides a use of the antibody or antigen-binding fragment thereof or the conjugate as described above in the manufacture of a detection reagent for detecting the presence or level of CD40 in a sample.

In certain embodiments, the detection reagent detects the presence or level of CD40 in the sample by the method as described above.

In certain embodiments, the sample is a cell sample (e.g., immune cell) from a subject (e.g., a mammal, preferably a human, or a cynomolgus monkey).

### Definition of Terms

In the present application, unless otherwise stated, scientific and technical terms used herein have the meanings commonly understood by those skilled in the art. Moreover, the virology, biochemistry, and immunology laboratory procedures used in herein are routine procedures widely used in the corresponding fields. Meanwhile, in order to better understand the present application, definitions and explanations of relevant terms are provided below.

When the terms "for example," "such as," "e.g.," "comprising," "including," or variations thereof are used herein, these terms will not be considered limiting terms and will instead be interpreted to mean "but without limitation" or "without limitation".

Unless otherwise indicated herein or clearly contradicted by context, the terms "a" and "an" as well as "the" and similar referents in the context for describing the present application (especially in the context of the following claims) are to be construed to cover singular and plural.

As used herein, the term "antibody" refers to an immunoglobulin molecule typically composed of two pairs of polypeptide chains, each pair having a light chain (LC) and a heavy chain (HC). Antibody light chains can be classified into k (kappa) and λ (lambda) light chains. Heavy chains can be classified as µ, δ, γ, α, or ε, and define the antibody's isotypes as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CHI, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin with host tissue or factor, including various cells of the immune system (e.g., effector cells) and the first component of the classical complement system (Clq). The VH and VL regions can also be subdivided into regions of high variability called complementarity-determining regions (CDRs), interspersed with more conservative regions called framework regions (FRs). Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form an antigen-binding site. The assignment of amino acids to regions or domains can follow the definition as described in Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity-determining region" or "CDR" refers to those amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5 th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present application, the CDRs contained in the antibodies of the present invention or antigen-binding fragments thereof may be determined according to various numbering systems known in the art. In certain embodiments, the CDRs contained in the antibodies of the present invention or antigen-binding fragments thereof are determined by the Kabat, Chothia, or IMGT numbering systems. In certain embodiments, the CDRs contained in the antibodies of the present invention or antigen-binding fragments thereof are determined by the numbering method as described in the "Sequence analysis" section on page 11 of Lu X, Nobrega RP, Lynaugh H, et al. Deamidation and isomerization liability analysis of 131 clinical-stage antibodies. MAbs. 2019 Jan;11(1):45-57. doi: 10.1080/19420862.2018.1548233, which is incorporated herein by reference in their entirety; wherein CDRs L1-L3 refer to positions 24-34, 50-56, 89-97, respectively, and CDRs H1-H3 refer to positions 27-35, 50-65, and 93-102, respectively; wherein said positions refer to the positions of Kabat.

As used herein, the term "framework region" or "FR" residues refers to those amino acid residues in an antibody variable region other than the CDR residues as defined above.

The term "antibody" is not limited to any particular method of producing the antibody. It includes, for example, recombinant antibody, monoclonal antibody, and polyclonal antibody. The antibody may be of different isotypes, for example, IgG (e.g., IgGl, IgG2, IgG3 or IgG4 subtypes), IgAl, IgA2, IgD, IgE or IgM antibody.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of an antibody can be obtained by recombinant DNA technology or by enzymatic or chemical cleavage of an intact antibody. Non-limiting examples of antigen-binding fragments include Fab, Fab', F(ab')₂, Fd, Fv, complementarity-determining region (CDR) fragment, scFv, diabody, single domain antibody, chimeric antibody, linear antibody, nanobody (technology from Domantis), probody, and such polypeptides, which contain at least a portion of an antibody that is sufficient to confer a specific antigen-binding capability. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains." Among them, "full-length heavy chain" refers to a polypeptide chain that consists of a heavy chain variable region (VH), a heavy chain constant region CH1 domain, a hinge region (HR), a heavy chain constant region CH2 domain, a heavy chain constant region CH3 domain in the direction from the N-terminal to the C-terminal; and, when the full-length antibody is of IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and between the HRs of the two full-length heavy chains. The full-length antibody of the present invention can be from a single species, such as human; it can also be a chimeric antibody or a humanized antibody. The full-length antibody of the present invention contains two antigen-binding sites formed by VH and VL pairs respectively, and these two antigen-binding sites specifically recognize/bind the same antigen.

As used herein, the term "Fd" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')₂ fragment" refers to an antibody fragment consisting of two Fab fragments connected by disulfide bridges on the hinge region; the term "Fab' fragment" refers to a fragment obtained by reducing the disulfide bond connecting the two heavy chain fragments in the F(ab')₂ fragment, and consists of a complete light chain and heavy chain Fd fragment (consisting of VH and CH1 domains).

As used herein, the term "Fv" refers to an antibody fragment consisting of VL and VH domains of a single arm of an antibody. Fv fragments are generally considered to be the smallest antibody fragments that can form a complete antigen-binding site. It is generally believed that six CDRs confer the antigen-binding specificity of an antibody. However, even a variable region (e.g., an Fd fragment, which contains only three antigen-specific CDRs) can recognize and bind the antigen, although its affinity may be lower than that of the intact binding site.

As used herein, the term "Fc" refers to an antibody fragment formed by bonding of the second and third constant regions of a first heavy chain of an antibody to the second and third constant regions of a second heavy chain of an antibody via disulfide bonds. The Fc fragment of an antibody has many different functions but does not participate in antigen binding.

As used herein, the term "scFv" refers to a single polypeptide chain comprising VL and VH domains, in which the VL and VH are connected via a linker (see, for example, Bird et al., Science.242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH₂-VL-linker-VH-COOH or NH₂-VH-linker-VL-COOH. Suitable prior art linkers consist of repeated GGGGS amino acid sequences or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers useful in the present application are described in Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol. In some cases, a disulfide bond may also exist between VH and VL of scFv. In certain embodiments of the present application, scFv can form di-scFv, which refers to an antibody formed by connecting two or more individual scFvs in series. In certain embodiments of the present application, scFv can form (scFv)₂, which refers to an antibody formed by connecting two or more individual scFvs in parallel.

As used herein, the term "diabody" refers to an antibody whose VH and VL domains are expressed on a single polypeptide chain, but the linker used is too short to allow pairing between the two domains on the same chain, which forces the domains to pair with the complementary domains of the other chains and generate two antigen-binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the common meaning as generally understood by those skilled in the art, and refers to an antibody fragment consisting of a single monomeric variable antibody domain (e.g., a single heavy chain variable region), which retains the ability to specifically bind to the same antigen to which the full-length antibody binds. The single domain antibody is also called nanobody.

Each of the above antibody fragments retains the ability to specifically bind to the same antigen to which the full-length antibody binds, and/or competes with the full-length antibody for specific binding to the antigen.

Antigen-binding fragments of antibody (e.g., the above-described antibody fragments) can be obtained from a given antibody (e.g., the antibody provided by the present application) using conventional techniques known to those skilled in the art (e.g., recombinant DNA technology or enzymatic or chemical fragmentation methods), and the antigen-binding fragments of the antibody are screened for specificity in the same manner as for intact antibodies.

As used herein, the term "chimeric antibody" refers to an antibody in which part of the light chain and/or heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), nevertheless, it still retains the binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include such an antibody in which the heavy and light chain variable regions of the antibody are derived from a first antibody and the heavy and light chain constant regions of the antibody are derived from a second antibody.

As used herein, the term "identity" is used to refer to the match of sequences between two polypeptides or between two nucleic acids. To determine the percent identity of two amino acid sequences or two nucleic acid sequences, the sequences are aligned for optimal comparison purposes (e.g., gaps may be introduced in the first amino acid sequence or nucleic acid sequence for best alignment with the second amino acid or nucleic acid sequence). The amino acid residues or nucleotides at the corresponding amino acid positions or nucleotide positions are then compared. Molecules are identical at a position when the position in the first sequence is occupied by the same amino acid residue or nucleotide as the corresponding position in the second sequence. The percent identity between two sequences is a function of the number of identical positions shared by the sequences (i.e., percent identity = number of identical overlapping positions/total number of positions × 100%). In certain embodiments, both sequences are of the same length.

Determination of percent identity between two sequences can also be accomplished using mathematical algorithms. One non-limiting example of mathematical algorithm for comparison of two sequences is the algorithm of Karlin and Altschul, 1990, Proc. Natl. Acad. Sci. U.S.A. 87:2264-2268, which was improved by Karlin and Altschul, 1993, Proc. Natl. Acad. Sci. U.S.A. 90:5873-5877. Such algorithms were integrated into the NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by introducing a substitution, deletion, or addition of amino acid residues. In some cases, the term "variant" also refers to a polypeptide or peptide that has been modified (i.e., by covalently linking any type of molecule to the polypeptide or peptide). For example, and without limitation, polypeptides may be modified, for example, by glycosylation, acetylation, PEGylation, phosphorylation, amidation, derivatization by known protecting/blocking groups, proteolytic cleavage, attachment to cellular ligand or other proteins, etc. Derivatized polypeptides or peptides can be produced by chemical modification using techniques known to those skilled in the art, including, but not limited to, specific chemical cleavage, acetylation, formylation, metabolic synthesis of tunicamycin, and the like. Furthermore, a variant has a similar, identical or improved function to the polypeptide or peptide from which it is derived.

As used herein, the term "specific binding" refers to a non-random binding reaction between two molecules, such as the reaction between an antibody and the antigen it targets. The strength or affinity of a specific binding interaction can be expressed by the equilibrium dissociation constant (K_{D}) of the interaction. In the present application, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding properties between two molecules can be determined using methods known in the art. One approach involves measuring the rate at which antigen binding site/antigen complex forms and dissociates. Both the "association rate constant" (kₐ or kₒₙ) and the "dissociation rate constant" (k_{dis} or k_{off}) can be calculated from concentrations and actual rates of association and dissociation (see Malmqvist M, Nature, 1993, 361 :186-187). The ratio k_{dis}/kₒₙ is equal to the dissociation constant K_{D} (see Davies et al., Annual Rev Biochem, 1990; 59:439-473). K_{D}, kₒₙ and k_{dis} values can be measured by any valid method. In certain embodiments, dissociation constants can be measured by surface plasmon resonance (SPR) with a Biacore instrument. Besides, bioluminescence interferometry or Kinexa can be used to measure dissociation constants.

As used herein, a detectable label of the present application may be any substance detectable by fluorescent, spectroscopic, photochemical, biochemical, immunological, electrical, optical or chemical means. Such labels are well known in the art and examples include, but are not limited to, enzymes (e.g., horseradish peroxidase, alkaline phosphatase, beta-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g., Cy7, Alexa 750)), luminescent substances (e.g., chemiluminescent substances, such as acridinium esters, luminol and its derivatives compounds, ruthenium derivatives such as ruthenium terpyridyl), magnetic beads (e.g., Dynabeads^{®}), calorimetric markers such as colloidal gold or colored glass or plastic (e.g., polystyrene, polypropylene, latex, etc.) beads, and biotin for binding to avidin (e.g., streptavidin) modified with the above label.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or Pl-derived artificial chromosome (PAC); phage, such as λ phage or M13 phage and animal virus, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can contain a variety of expression-controlling elements, including, but not limited to, promoter sequence, transcription initiation sequence, enhancer sequence, selection element, and reporter gene. In addition, the vector may also contain an origin of replication.

As used herein, the term "host cell" refers to a cell that can be used to introduce a vector, which includes, but is not limited to, prokaryotic cell such as *E. coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutation. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which is incorporated herein by reference).

The twenty conventional amino acids involved herein have been written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present application, the terms "polypeptide" and "protein" have the same meaning and are used interchangeably. And in the present application, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, being not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes sterile injectable liquid (e.g., aqueous or non-aqueous suspensions or solutions). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

As used herein, the term "prevention" refers to a method performed to prevent or delay the occurrence of a disease or condition or symptom in a subject. As used herein, the term "treatment" refers to a method performed to obtain a beneficial or desired clinical outcome. For the purposes of this application, beneficial or desired clinical outcomes include, but are not limited to, alleviation of symptoms, reduction of the extent of disease, stabilization (i.e., no worsening) of the state of disease, delaying or slowing the progression of disease, ameliorating or alleviating the status of disease, and relief of symptoms (whether partial or complete), whether detectable or undetectable. In addition, "treatment" may also refer to prolonging survival compared to expected survival if not receiving treatment.

As used herein, the term "subject" refers to a mammal, such as a human. In certain embodiments, the subject (e.g., a human) has a tumor, an infection or an autoimmune disease, or is at a risk of suffering from the aforementioned disease.

As used herein, the term "effective amount" refers to an amount sufficient to achieve, or at least partially achieve, the desired effect. For example, an effective amount for preventing a disease (e.g., a tumor, an infection or an autoimmune disease) refers to an amount that is sufficient to prevent, arrest or delay the occurrence of the disease; the effective amount for treating a disease refers to an amount that is sufficient to cure or at least partially prevent the disease and its complications in a patient who already has the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently, and so on.

### Beneficial Effects of the present invention

The present application provides fully human antibodies against CD40, which have high binding affinity to CD40, exhibit cross-reactivity with human and cynomolgus CD40, and can either block or non-block the binding of CD40 to its ligand CD40L.

### Brief Description of the Drawings

Fig. 1 shows the binding activity of the parent anti-CD40 candidate molecule to CHO cells expressing human CD40.
Fig. 2 shows the activity of parent anti-CD40 candidate molecule in activating the reporter gene for CD40 signaling in the absence of beads cross-linking.
Fig. 3 shows the activity of the parent anti-CD40 candidate molecule in activating the reporter gene for CD40 signaling in the presence of beads cross-linking.
Fig. 4 shows the binding activity of the progeny anti-CD40 candidate molecules to CHO cells expressing human CD40.
Fig. 5 shows the binding activity of the progeny anti-CD40 candidate molecules to CHO cells expressing cynomolgus CD40.
Fig. 6 shows the activity of progeny anti-CD40 candidate molecules in activating the reporter gene for CD40 signaling in the presence of anti-Fc cross-linking.
Fig. 7 shows the activity of progency anti-CD40 candidate molecules in activating the reporter gene for CD40 signaling in the absence of anti-Fc cross-linking.

The embodiments of the present invention will be described in detail below with reference to the accompanying drawings and examples, but those skilled in the art will understand that the following drawings and examples are only used to illustrate the present invention and do not limit the scope of the present invention. Various objects and advantageous aspects of the present invention will become apparent to those skilled in the art from the accompanying drawings and the following detailed description of preferred embodiments.

### Sequence Information

A description of the sequences covered by the present application is provided in the table below.

**Table 1: Description of sequences**

| SEQ ID NO: | Sequence Description |
|---|---|
| 1-6, 12-70, 82-83 | Sequences of the light and heavy chain variable regions and CDR regions of each antibody, as shown in Table 3 |
| 71 | Human CD40 amino acid sequence |
| | MVRLPLQCVLWGCLLTAVHPEPPTACREKQYLINSQCCSLCQPGQKLVS DCTEFTETECLPCGESEFLDTWNRETHCHQHKYCDPNLGLRVQQKGTSE TDTICTCEEGWHCTSEACESCVLHRSCSPGFGVKQIATGVSDTICEPCPV GFFSNVSSAFEKCHPWTSCETKDLVVQQAGTNKTDVVCGPQDRLRALV VIPIIFGILFAILLVLVFIKKVAKKPTNKAPHPKQEPQEINFPDDLPGSNTA APVQETLHGCQPVTQEDGKESRISVQERQ |
| 72 | Amino acid sequence of heavy chain constant region of the antibody |
| | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSRDELTKN QVSLTCLVKGFYPSDIAVEWESNGQPENNYKTTPPVLDSDGSFFLYSKL TVDKSRWQQGNVFSCSVMHEALHNHYTQKSLSLSPG |
| 73 | Amino acid sequence of light chain constant region of the antibody |
| | |
| 74 | Amino acid sequence of cynomolgus CD40 |
| | MVSKGEELFTGVVPILVELDGDVNGHKFSVSGEGEGDATYGKLTLKFIC TTGKLPVPWPTLVTTLTYGVQCFSRYPDHMKKHDFFKSAMPEGYVQER TIFFKDDGNYKTRAEVKFEGDTLVNRIELKGIDFKEDGNILGHKLEYNY NSHNVYIMADKQKNGIKANFKVRHNIEDGSVQLADHYQQNTPIGDGPV LLPDNHYLSTQSALSKDPNEKRDHMVLLEFVTAAGITLGMDELYK |
| 75 | Amino acid sequence of human CD40L |
| | |
| 76 | FTFX₁X₂YX₃MH |
| 77 | VIX₄YX₅X₆X₇X₈KYYAX₉SVKG |
| 78 | GISWSSX₁₀SIX₁₁YADSVKG |
| 79 | X₁₂ASQSVSX₁₃X₁₄YLA |
| 7 | X₁₅ASX₁₆X₁₇ISSWLA |
| 8 | GASX₁₈X₁₉X₂₀X₂₁ |
| 9 | AASX₂₂LX₂₃S |
| 10 | X₂₄X₂₅YX₂₆DYPPFT |
| 11 | QQX₂₇X₂₈SX₂₉PPT |
| 80 | Amino Acid Sequence of the Light Chain of CP870893-IgG2 |
| | MRLPAQLLGLLLLWFPGSRCDIQMTQSPSSVSASVGDRVTITCRASQGIYSW LAWYQQKPGKAPNLLIYTASTLQSGVPSRFSGSGSGTDFTLTISSLQPEDFAT YYCQQANIFPLTFGGGTKVEIKRTVAAPSVFIFPPSDEQLKSGTASVVCLLNN FYPREAKVQWKVDNALQSGNSQESVTEQDSKDSTYSLSSTLTLSKADYEK HKVYACEVTHQGLSSPVTKSFNRGEC |
| 81 | Amino Acid Sequence of the heavy Chain of CP870893-IgG2 |
| | MDWTWRILFLVAAATGAHSQVQLVQSGAEVKKPGASVKVSCKASGYTFTG YYMHWVRQAPGQGLEWMGWINPDSGGTNYAQKFQGRVTMTRDTSISTAY MELNRLRSDDTAVYYCARDQPLGYCTNGVCSYFDYWGQGTLVTVSSAST KGPSVFPLAPCSRSTSESTAALGCLVKDYFPEPVTVSWNSGALTSGVHTFPA VLQSSGLYSLSSVVTVPSSNFGTQTYTCNVDHKPSNTKVDKTVERKCCVEC PPCPAPPVAGPSVFLFPPKPKDTLMISRTPEVTCVVVDVSHEDPEVQFNWYV DGVEVHNAKTKPREEQFNSTFRVVSVLTVVHQDWLNGKEYKCKVSNKGL PAPIEKTISKTKGQPREPQVYTLPPSREEMTKNQVSLTCLVKGFYPSDIAVEW ESNGQPENNYKTTPPMLDSDGSFFLYSKLTVDKSRWQQGNVFSCSVMHEA LHNHYTQKSLSLSPGK |

### Specific Models for Carrying Out the Invention

The present invention is now described with reference to the following examples which are intended to illustrate the present invention (but not to limit the present invention).

Unless otherwise specified, the molecular biology experimental methods and immunoassay methods used in the present application were carried out basically referring to the methods described by J. Sambrook et al., Molecular Cloning: A Laboratory Manual, 2nd Edition, Cold Spring Harbor Laboratory Press, 1989, and F. M. Ausubel et al., Compiled Laboratory Guide to Molecular Biology, 3rd Edition, John Wiley & Sons, Inc., 1995; the use of restriction enzymes was in accordance with the conditions recommended by the product manufacturer. Those skilled in the art will appreciate that the examples describe the present invention by way of example and are not intended to limit the scope of the present invention as claimed.

### Example 1: Preparation of Antibodies

### 1.1 Yeast Display Technology for Screening anti-CD40 Fully Human antibody

Based on the yeast display antibody library (Adimab), eight natural synthetic libraries were amplified according to existing methods (WO2009036379; WO2010105256; WO2012009568), with each library's diversity reaching 1×10⁹. In brief, the first two rounds of screening were performed using Miltenyi's MACS system for magnetic bead cell sorting. Initially, yeast cells from the library (~1×10¹⁰ cells per library) were incubated in FACS wash buffer (phosphate-buffered saline containing 0.1% BSA) at room temperature for 15 minutes, with the buffer containing 10 nM biotinylated human CD40-Fc fusion protein (purchased from ACRO, catalog number: CD0-H5253, wherein the amino acid sequence of human CD40 is shown as SEQ ID NO: 71). The cells were washed once with 50 mL of pre-chilled FACS wash buffer, then resuspended in 40 mL of buffer, and 500 µL of streptavidin magnetic beads (Miltenyi LS) were added, followed by incubation at room temperature for 15 minutes. After centrifugation at 1000rpm for 5 minutes to discard the supernatant, the cells were resuspended in 5 mL of FACS wash buffer, and the cell suspension was added to a Miltenyi LS column. After loading, the column was washed three times with 3 mL of FACS wash buffer each time. The Miltenyi LS column was removed from the magnetic field, and the elution was performed with 5 mL of growth medium, the eluted yeast cells were collected and allowed to grow overnight at 37°C.

For the next round of sorting using flow cytometry: The yeast cells obtained from the MACS system screening, approximately 1×10⁸, were then washed three times with FACS buffer and cultured at room temperature in a solution containing 100 nM biotinylated CD40 monomer protein (purchased from Acro, catalog number: CD0-H5228, wherein the amino acid sequence of the said CD40 is shown as SEQ ID NO: 71) or 10 nM CD40-Fc fusion protein. After discarding the culture medium, the cells were washed twice with FACS wash buffer, then mixed with LC-FITC (FITC-labeled anti-human immunoglobulin kappa light chain antibody, purchased from Southern Biotech) (diluted 1:100), and mixed with SA-633 (streptavidin-633, Molecular Probes) (diluted 1:500) or SA-PE (streptavidin-PE, Sigma) (diluted 1:50), and incubated at 4°C for 15 minutes. The cells were washed twice with pre-chilled FACS wash buffer and resuspended in 0.4 mL of buffer, then transferred to a separation tube with a filter. Cells were sorted using the FACS ARIA (BD Biosciences).

The yeast cells expressing anti-CD40 antibodies obtained through screening were induced to secrete anti-CD40 antibodies (full-length antibodies) by shaking at 30°C for 48 hours. After the induction was complete, the yeast cells were removed by centrifugation at 1300rpm for 10 minutes, and the supernatant was collected. The anti-CD40 antibodies in the supernatant were purified using Protein A, eluted with a pH 2.0 acetic acid solution, and the anti-CD40 antibodies were harvested. This screening yielded anti-CD40 antibodies ADI-47754, ADI-47720, and ADI-47741.

### 1.2 Affinity optimization of anti-human CD40 antibody

To obtain higher-affinity antibody, we optimized antibodies ADI-47754 and ADI-47720 through, but not limited to, the following methods.

### 1.3 CDRH1/CDRH2 screening

The CDRH3 gene of the parent molecule was constructed into a CDRH1/CDRH2 gene library with a diversity of 1×10⁸, and was subjected to four rounds of screening. The first two rounds used the MACS method, while the third and fourth rounds used the FACS method to apply affinity pressure on the antibody-antigen complexes, screening for high-affinity antibodies.

The yeast cells expressing anti-CD40 antibodies, obtained from the screening, were cultured at 30°C with shaking for 48 hours to produce CD40 antibodies. After the induction of expression was complete, the yeast cells were removed by centrifugation at 1300rpm for 10 minutes, and the supernatant was collected. The anti-CD40 antibodies in the supernatant were purified using Protein A, and eluted with a pH 2.0 acetic acid solution to harvest the anti-CD40 antibodies. The corresponding Fab fragments were obtained by using papain and purified with KappaSelect (GE Healthcare).

### 1.4 VHmut Screening

In this method, a mutation was introduced into the heavy chain region through conventional error-prone PCR. During the PCR process, the base mismatch probability is increased to about 0.01 bp by using 1 µM of highly mutagenic base analogs dPTP and 8-oxo-dGTP.

The products obtained through error-prone PCR are constructed into a vector containing the heavy chain constant region by homologous recombination. Using this method, a secondary library with a capacity of 1×10⁷ was obtained under selection pressures including CD40 antigen titers, unlabeled antigen competition, and competition using the parental antibody. The library was then subjected to three rounds of screening using the FACS method, and the yeast cells expressing the corresponding antibodies were obtained.

After affinity maturation, the relationship between each antibody progeny and the parental antibody is shown in Table 2.

**Table 2. the Relationship between the Anti-CD40 Molecule Progeny and Parental**

| Parental Clone No. | Progeny Clone No. |
|---|---|
| ADI-47754 | ADI-55136; ADI-55140; ADI-55142; ADI-55144; ADI-55144-2; ADI-55145; ADI-55147 |
| ADI-47720 | ADI-55164; ADI-55168 |

### 1.5 Expression and Purification of Antibody

The sequences and designations of various anti-human CD40 antibody clones involved in this application are shown in Table 3, which includes the parental antibody ADI-47741 not mentioned in Table 2.

Antibodies used in the examples were expressed by HEK293 cells and purified. The certain operation is as follows: the pcDNA3.1 vector carrying the encoding sequences of heavy and light chains of the antibody was transfected into HEK293 cells using a chemical transfection method (wherein, the amino acid sequence of the heavy chain constant region encoded by the vector is shown as SEQ ID NO: 72, and the amino acid sequence of the light chain constant region is shown as SEQ ID NO: 73), and cultured under conditions of 37°C and 8% CO₂ for 7 days. The cell culture was collected and centrifuged at 13,000 rpm for 20 minutes. The supernatant was taken, and the antibodies were purified with Protein A. The purity of the antibodies was detected by SEC, and the endotoxin content was controlled simultaneously.

**Table 3. Sequence information of the variable regions and CDR regions of heavy and light chains of anti-CD40 clones**

| Clone NO. | VH CDR1 | VH CDR2 | VH CDR3 | VH | VL CDR1 | VL CDR2 | VL CDR3 | VL |
|---|---|---|---|---|---|---|---|---|
| ADI-47754 | FTFSS YGMH | VISYE GSNK YYADS VKG | ARDTS RGHDI | QVQLVESGGGVVQPGRSLR LSCAASGFTFSSYGMHWVR QAPGKGLEWVAVISYEGSN KYYADSVKGRFTISRDNSK NTLYLQMNSLRAEDTAVYY CARDTSRGHDIWGQGTMV TVSS | RASQS VSSSY LA | GASSR AT | QQYA DYPPF T | EIVLTQSPGTLSLSPGERATL SCRASQSVSSSYLAWYQQK PGQAPRLLIYGASSRATGIPD RFSGSGSGTDFTLTISRLEPE DFAVYYCQQYADYPPFTFG GGTKVEIK |
| SEQ ID NO. | 1 | 12 | 23 | 26 | 37 | 45 | 54 | 61 |
| ADI-55136 | FTFSS YGMH | VISYH ASSKY YADSV KG | ARDTS RGHDI | QVQLVESGGGVVQPGRSLR LSCAASGFTFSSYGMHWVR QAPGKGLEWVAVISYHASS KYYADSVKGRFTISRDNSK NTLYLQMNSLRAEDTAVYY CARDTSRGHDIWGQGTMV TVSS | RASQS VSSSY LA | GASSR AT | QQYA DYPPF T | EIVLTQSPGTLSLSPGERATL SCRASQSVSSSYLAWYQQK PGQAPRLLIYGASSRATGIPD RFSGSGSGTDFTLTISRLEPE DFAVYYCQQYADYPPFTFG GGTKVEIK |
| SEQ ID NO. | 1 | 13 | 23 | 27 | 37 | 45 | 54 | 61 |
| ADI-55140 | FTFEK YGMH | VIHYE GSIKY YADSV KG | ARDTS RGHDI | QVQLVESGGGVVQPGRSLR LSCAASGFTFEKYGMHWVR QAPGKGLEWVAVIHYEGSIK YYADSVKGRFTISRDNSKNT LYLQMNSLRAEDTAVYYCA RDTSRGHDIWGQGTMVTVS S | RASQS VSYD YLA | GASTR AN | GEYA DYPPF T | EIVLTQSPGTLSLSPGERATL SCRASQSVSYDLAWYQQK PGQAPRLLIYGASTRANGIP DRFSGSGSGTDFTLTISRLEP EDFAVYYCGEYADYPPFTFG GGTKVEIK |
| SEQ ID NO. | 2 | 14 | 23 | 28 | 38 | 46 | 55 | 62 |
| ADI-55142 | FTFEK YGMH | VISYE GEKK YYADS VKG | ARDTS RGHDI | QVQLVESGGGVVQPGRSLR LSCAASGFTFEKYGMHWVR QAPGKGLEWVAVISYEGEK KYYADSVKGRFTISRDNSK NTLYLQMNSLRAEDTAVYY CARDTSRGHDIWGQGTMV TVSS | RASQS VSYSY LA | GASSR AN | SDYAD YPPFT | EIVLTQSPGTLSLSPGERATL SCRASQSVSYSYLAWYQQK PGQAPRLLIYGASSRANGIP DRFSGSGSGTDFTLTISRLEP EDFAVYYCSDYADYPPFTFG GGTKVEIK |
| SEQ ID NO. | 2 | 15 | 23 | 29 | 39 | 47 | 56 | 63 |
| ADI-55144 | FTFEK YGMH | VISYE GTKK YYADS VKG | ARDTS RGHDI | QVQLVESGGGVVQPGRSLR LSCAASGFTFEKYGMHWVR QAPGKGLEWVAVISYEGTK KYYADSVKGRFTISRDNSK NTLYLQMNSLRAEDTAVYY CARDTSRGHDIWGQGTMV TVSS | RASQS VSYSY LA | GASSL AT | QQYS DYPPF T | EIVLTQSPGTLSLSPGERATL SCRASQSVSYSYLAWYQQK PGQAPRLLIYGASSLATGIPD RFSGSGSGTDFTLTISRLEPE DFAVYYCQQYSDYPPFTFG GGTKVEIK |
| SEQ ID NO. | 2 | 16 | 23 | 30 | 39 | 48 | 57 | 64 |
| ADI-55145 | FTFEK YGMH | VISYE GTSKY YADSV KG | ARDTS RGHDI | QVQLVESGGGVVQPGRSLR LSCAASGFTFEKYGMHWVR QAPGKGLEWVAVISYEGTS KYYADSVKGRFTISRDNSK NTLYLQMNSLRAEDTAVYY CARDTSRGHDIWGQGTMV TVSS | QASQS VSYN YLA | GASSR NN | QQYS DYPPF T | EIVLTQSPGTLSLSPGERATL SCQASQSVSYNYLAWYQQK PGQAPRLLIYGASSRNNGIP DRFSGSGSGTDFTLTISRLEP EDFAVYYCQQYSDYPPFTFG GGTKVEIK |
| SEQ ID NO. | 2 | 17 | 23 | 31 | 40 | 49 | 57 | 65 |
| ADI-55147 | FTFGS YGMH | VISYE GVKK YYADS VKG | ARDTS RGHDI | QVQLVESGGGVVQPGRSLR LSCAASGFTFGSYGMHWVR QAPGKGLEWVAVISYEGVK KYYADSVKGRFTISRDNSK NTLYLQMNSLRAEDTAVYY CARDTSRGHDIWGQGTMV TVSS | RASQS VSYD YLA | GASA RAN | QQYA DYPPF T | EIVLTQSPGTLSLSPGERATL SCRASQSVSYDYLAWYQQK PGQAPRLLIYGASARANGIP DRFSGSGSGTDFTLTISRLEP EDFAVYYCQQYADYPPFTFG GGTKVEIK |
| SEQ ID NO. | 3 | 18 | 23 | 32 | 38 | 50 | 54 | 66 |
| ADI-47720 | FTFDD YAMH | GISWS SGSIG YADSV KG | AKDPT ATTGA RGYF DL DL | EVQLVESGGGLVQPGRSLRL SCAASGFTFDDYAMHWVR QAPGKGLEWVSGISWSSGSI GYADSVKGRFTISRDNAKN SLYLQMNSLRAEDTAVYYC AKDPTATTGARGYFDLWGR GTLVTVSS | RASQ GISSW LA | AASSL QS | QQRA SFPPT | DIQMTQSPSSVSASVGDRVT ITCRASQGISSWLAWYQQKP GKAPKLLIYAASSLQSGVPS RFSGSGSGTDFTLTISSLQPE DFATYYCQQRASFPPTFGGG TKVEIK |
| SEQ ID NO. | 4 | 19 | 24 | 33 | 41 | 51 | 58 | 67 |
| ADI-55164 | FTFDD YAMH | GISWS SDSIH YADSV KG | AKDPT ATTGA RGYF DL | EVQLVESGGGLVQPGRSLRL SCAASGFTFDDYAMHWVR QAPGKGLEWVSGISWSSDSI HYADSVKGRFTISRDNAKN SLYLQMNSLRAEDTAVYYC AKDPTATTGARGYFDLWGR GTLVTVSS | RASK VISSW LA | AASYL ES | QQHSS FPPT | DIQMTQSPSSVSASVGDRVT ITCRASKVISSWLAWYQQKP GKAPKLLIYAASYLESGVPS RFSGSGSGTDFTLTISSLQPE DFATYYCQQHSSFPPTFGGG TKVEIK |
| SEQ ID NO. | 4 | 20 | 24 | 34 | 42 | 52 | 59 | 68 |
| ADI-55168 | FTFDT YAMH | GISWS SGSIH YADSV KG | AKDPT ATTGA [?]GYF DL | EVQLVESGGGLVQPGRSLRL SCAASGFTFDTYAMHWVRQ APGKGLEWVSGISWSSGSIH YADSVKGRFTISRDNAKNSL YLQMNSLRAEDTAVYYCAK DPTATTGARGYFDLWGRGT LVTVSS | EASQV ISSWL A | AASYL QS | QQHSS FPPT | DIQMTQSPSSVSASVGDRVT ITCEASQVISSWLAWYQQKP GKAPKLLIYAASYLQSGVPS RFSGSGSGTDFTLTISSLQPE DFATYYCQQHSSFPPTFGGG TKVEIK |
| SEQ ID NO. | 5 | 21 | 24 | 35 | 43 | 53 | 59 | 69 |
| ADI-47741 | GSISS YYWS | SIYYS GSTNY NPSLK S | ARDA NYYK WHPY | QVQLQESGPGLVKPSETLSL TCTVSGGSISSYYWSWIRQP PGKGLEWIGSIYYSGSTNYN PSLKSRVTISVDTSKNQFSLK LSSVTAADTAVYYCARDAN YYKWHPYWGQGTLVTVSS | RASQS VSSDY LA | GASSR AT | QQVLS YPPT | EIVLTQSPGTLSLSPGERATL SCRASQSVSSDYLAWYQQK PGQAPRLLIYGASSRATGIPD RFSGSGSGTDFTLTISRLEPE DFAVYYCQQVLSYPPTFGG GTKVEIK |
| SEQ ID NO. | 6 | 22 | 25 | 36 | 44 | 45 | 60 | 70 |
| ADI-55144-2 | FTFEK YGMH | VISYE GTKK YYAES VKG | ARDTS RGHDI | QVQLVESGGGVVQPGRSLR LSCAASGFTFEKYGMHWVR QAPGKGLEWVAVISYEGTK KYYAESVKGRFTISRDNSKN TLYLQMNSLRAEDTAVYYC ARDTSRGHDIWGQGTMVT VSS | RASQS VSYSY LA | GASSL AT | QQYS DYPPF T | EIVLTQSPGTLSLSPGERATL SCRASQSVSYSYLAWYQQK PGQAPRLLIYGASSLATGIPD RFSGSGSGTDFTLTISRLEPE DFAVYYCQQYSDYPPFTFG GGTKVEIK |
| SEQ ID NO. | 2 | 82 | 23 | 83 | 39 | 48 | 57 | 64 |

### Example 2: Antibody Affinity Detection

The binding and dissociation constants (K_{D}) of the antibodies involved in this application for binding to human and cynomolgus CD40, as well as whether they block the binding of human CD40 to its ligand (human CD40L), were determined using the optical biosensor interference technology (ForteBio). The amino acid sequence of human CD40 is shown as SEQ ID NO: 71, the amino acid sequence of cynomolgus CD40 is shown as SEQ ID NO: 74, and the amino acid sequence of human CD40L is shown as SEQ ID NO: 75. The Fortebio affinity assay was conducted according to existing methods (Este, P et al. High throughput solution-based measurement of antibody-antigen affinity and epitope binning. Mabs, 2013.5(2):p.270-8).

Measuring the monovalent affinity of the candidate antibody Fab fragments to human and cynomolgus CD40-Fc proteins: The sensor was equilibrated offline in the analysis buffer for 20 minutes, followed by online detection for 120 seconds to establish a baseline, and then CD40-Fc antigen was loaded onto the AHQ sensor to a thickness of 1 nm for affinity detection. The antigen-loaded sensor was incubated with 100 nM Fab solution until a plateau was reached, and then the sensor was transferred to the analysis buffer for at least 2 minutes for dissociation rate measurement. Kinetic analysis was performed using a 1:1 binding model.

Measuring the bivalent affinity of intact antibodies to human and cynomolgus CD40-Fc proteins: The sensor was equilibrated offline in the analysis buffer for 20 minutes, then online detection for 120 seconds was used to establish a baseline, and the intact CD40 antibody was loaded onto the AHQ sensor to a thickness of 1 nm for affinity detection. The antibody-loaded sensor was further incubated in a high concentration of irrelevant intact antibody solution for 10 minutes to saturate and block the Fc binding sites on the AHQ sensor. The blocked sensor was then incubated with 100 nM CD40-Fc antigen until a plateau was reached, and then the sensor was transferred to the analysis buffer for at least 2 minutes for dissociation rate measurement. Kinetic analysis was performed using a 1:1 binding model.

In the aforementioned measurement method, the K_{D} values for each parental antibody molecule and its variant molecules are shown in Table 4:
From the results shown in the charts and tables, it can be seen that: (1) after affinity maturation, the monovalent affinity of each progeny molecule with human CD40 protein has significantly improved compared to the corresponding parental molecules. (2) after affinity maturation, the monovalent affinity of each progeny molecule with cynomolgus CD40 protein has significantly improved compared to the corresponding parental molecules.

**Table 4. K_{D} values of various parental antibody molecules and their variant molecules**

| Antibody | The bivalent affinity of IgG to human CD40-Fc protein | The monovalent affinity of Fab to human CD40-Fc protein | The bivalent affinity of IgG to cynomolgus CD40-Fc protein | The monovalent affinity of Fab to cynomolgus CD40-Fc protein | Whether to block the binding between human CD40 and its ligand, human CD40L |
|---|---|---|---|---|---|
| ADI-47754 | 8.27E-10 | 1.53E-07 | 1.04E-09 | 1.81E-07 | Yes |
| ADI-55136 | 2.97E-10 | 6.71E-08 | 3.83E-10 | 7.81E-08 | Yes |
| ADI-55140 | 1.68E-10 | 5.77E-09 | 2.30E-10 | 5.42E-09 | Yes |
| ADI-55142 | 1.46E-10 | 2.74E-09 | 1.93E-10 | 2.81E-09 | Yes |
| ADI-55144 | 1.10E-10 | 2.08E-09 | 1.90E-10 | 2.21E-09 | Yes |
| ADI-55145 | 1.69E-10 | 5.88E-09 | 2.26E-10 | 5.72E-09 | Yes |
| ADI-55147 | 1.39E-10 | 8.67E-09 | 2.08E-10 | 8.87E-09 | Yes |
| ADI-47720 | 9.32E-09 | 1.52E-07 | 6.07E-09 | 2.63E-07 | No |
| ADI-55164 | 1.00E-10 | 6.63E-10 | 1.17E-10 | 7.20E-10 | No |
| ADI-55168 | 1.11E-10 | 9.88E-10 | 1.22E-10 | 5.86E-10 | No |
| ADI-47741 | 3.56E-10 | 6.69E-08 | 5.59E-10 | 7.10E-08 | Yes |

| | | | | | |
|---|---|---|---|---|---|
| Note: P.F. = Poor Fit (poor fitting), N.B. = Non-Binder under the conditions of this assay(no binding detected in this test), N.A. = Not Available (not tested). | | | | | |

### Example 3: The Binding of Parental Anti-CD40 Antibody to Human CD40 Overexpressing CHO Cells

The binding activity of the parental anti-CD40 antibody with human CD40 expressing CHO cells was detected by flow cytometry. In this assay, the light and heavy chain amino acid sequences of the control antibody CP870893-IgG2 are shown as SEQ ID NOs: 80 and 81, respectively.

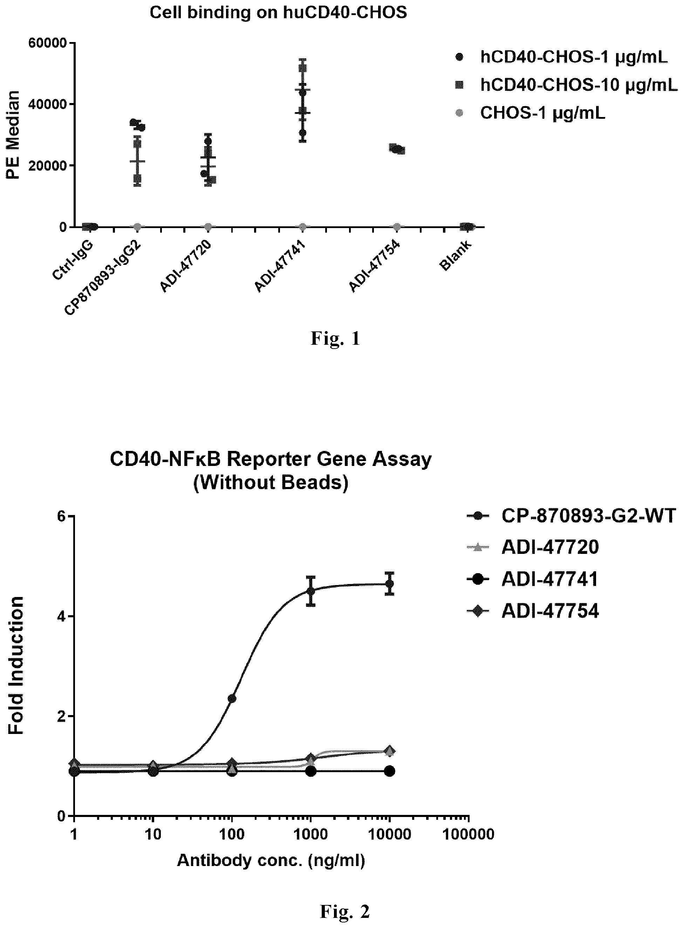

Specifically, by transfecting the pCHO1.0 vector (Invitrogen) with the human CD40 cDNA cloned into the MCS and followed by applying pressure selection, CHO-S cells overexpressing human CD40 (CHO-huCD40 cells) were generated. The expanded human CD40 overexpressing cells were adjusted to the appropriate cell density and plated on 96-well flow plate. After centrifugation, the cells were incubated with a gradient-diluted sample at 4°C for 30 minutes. The cells were then washed twice with PBS, followed by incubation with a fluorescent secondary antibody diluted to the appropriate concentration, at 4° C for 30 min. Following two washes with PBS, cells were resuspended in PBS and fluorescent signals were detection on the CytoFlex flow cytometer to calculate the corresponding MFI. The results are shown in Fig. 1.

The following conclusions can drawn from the experimental results: The parental anti-CD40 antibodies ADI-47754, ADI-47720, and ADI-47741 all exhibit good monovalent binding activity to CHO cells overexpressing human CD40.

### Example 4: Activation of Human CD40 Signaling Pathway by Parental Anti-CD40 Antibodies

The activity of the parental anti-CD40 antibodies in activating the CD40 signaling pathway with or without crosslinking by Protein G Magnetic Beads, was detected using a NFkB-Luc2P reporter gene assay.,
Specifically, U2OS cells overexpressing human CD40 were transfected with the NFkB-Luc2P vector (Promega, catalog number: 11501ES03) and followed by applying pressure selection, to generate NFkB-Luc2P-U2OS cells. Parental antibodies were added to NFkB-Luc2P-U2OS cells at final concentrations of 10 µg/ml, 1 µg/ml, 0.1 µg/ml, 0.01 µg/ml, and 0.001 µg/ml, along with 0.25 µL/well of Pierce^{™} Protein G Magnetic Beads (Thermo Scientific, catalog number: 88847). The cells were then incubated in the cell culture incubator for 4 h, to examine the CD40 downstream signaling activation induced by the antibody with the presence of bead crosslinking. Additionally, antibodies at the corresponding concentrations were added to NFkB-Luc2P-U2OS cells and incubated in a cell culture incubator for 4 hours to investigate the downstream signaling activation of CD40 induced by the antibody alone, without the presence of bead crosslinking.

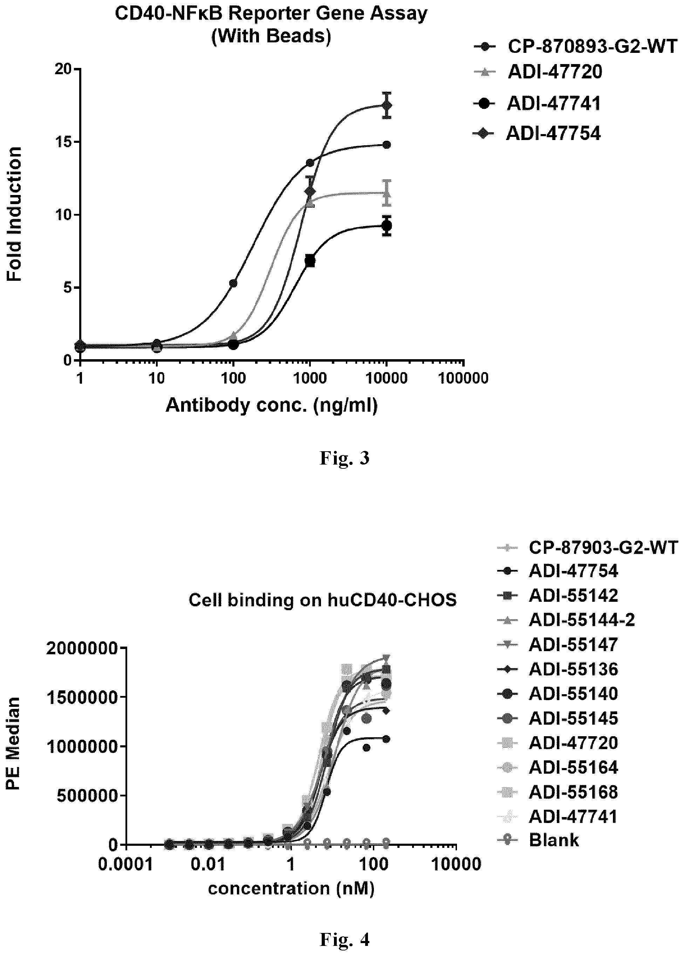

The following conclusions can drawn from the experimental results: Parental clones ADI-47754, ADI-47720, and ADI-47741 cannot activate the CD40 downstream signaling pathway without Fc crosslinking; with bead crosslinking, all clones can activate the CD40 downstream signaling pathway; and the signaling activity is enhanced in all antibodies with bead crosslinking. The positive control antibody CP-870893-G2-WT can activate the CD40 downstream signaling pathway independently of anti-Fc crosslinking. Detailed results can be found in Tables 5-7, and Figs. 2 and 3.

**Table 5. Activity of Parental Anti-CD40 Antibodies in Activating the CD40 Signaling Pathway**

| Antibodies | Signal amplification without bead crosslinking | Signal amplification with bead crosslinking |
|---|---|---|
| CP870893-IgG2-wt | 4.7 | 14.8 |
| ADI-47720 | 1.3 | 11.5 |
| ADI-47741 | 1.0 | 9.3 |
| ADI-47754 | 1.3 | 18.4 |

**Table 6. EC₅₀ values of the CD40-NFkB reporter gene assay (without beads cross-linking)**

| candidates | CP-870893-G2-WT | ADI-47720 | ADI-47741 | ADI-47754 |
|---|---|---|---|---|
| EC₅₀ (nM) | 131.1 | ~ 1071 | NA | 1494 |

**Table 7. EC₅₀ values of the CD40-NFkB reporter gene assay (with beads cross-linking)**

| Candidates | CP-870893-G2-WT | ADI-47720 | ADI-47741 | ADI-47754 |
|---|---|---|---|---|
| EC₅₀ (nM) | 181.1 | 303.6 | 639.3 | 767.9 |

### Example 5: Binding of Progeny Anti-CD40 Antibodies to Human/Cynomolgus CD40 expressing CHO Cells

Based on the results of Example 4, we optimized the affinity of ADI-47720, ADI-47741, and ADI-47754, which rely on bead crosslinking to activate the CD40 signaling, as reported by the reporter gene, resulting in the antibodies shown in Table 2.

Selected ADI-47720, ADI-47741, ADI-47754 and their progeny antibodies ADI-55136, ADI-55140, ADI-55142, ADI-55144-2, ADI-55145, ADI-55147, ADI-55164, ADI-55168 for the investigation of binding activity on human/cynomolgus CD40 overexpressing cells by Flow Cytometry.

Specifically, by transfecting the pCHO1.0 vector (Invitrogen) with the human/cynomolgus CD40 cDNA cloned into the MCS and followed by applying pressure selection, CHO-S cells overexpressing human/cynomolgus CD40 were generated. The expanded CD40 overexpressing cells were adjusted to the appropriate cell density and plated on 96-well flow plate. After centrifugation, the cells were incubated with a gradient-diluted sample at 4°C for 30 minutes. and the cells were then washed twice with PBS, and followed by incubation with a fluorescent secondary antibody diluted to the appropriate concentration, at 4° C for 30min. Following two washes with PBS, cells were resuspended in PBS and fluorescent signals were detection on the CytoFlex flow cytometer to calculate the corresponding MFI.

The following conclusions can drawn from the experimental results: Compared to the parental antibodies, the binding activity of the progeny anti-CD40 antibodies to human/ cynomolgus CD40-overexpressing cells has been improved to some extent, as shown in Tables 8-9 and Figs. 4-5.

**Table 8. EC₅₀ values for human CD40 expressing CHO Cell Binding Assay**

| Candidates | EC₅₀ (nM) |
|---|---|
| ADI-47754 | 7.005 |
| ADI-55142 | 7.689 |
| ADI-55144-2 | 11.49 |
| ADI-55147 | 7.493 |
| ADI-55136 | 4.988 |
| ADI-55140 | 6.3 |
| CP-870893 | 7.997 |
| ADI-55145 | 5.263 |
| ADI-47720 | 4.817 |
| ADI-55164 | 5.475 |
| ADI-55168 | 4.729 |
| ADI-47741 | 9.289 |

**Table 9. EC₅₀ values for cynomolgus CD40 expressing CHO Cell Binding Assay**

| Candidates | EC₅₀ (nM) |
|---|---|
| ADI-47754 | 5.794 |
| ADI-55142 | 6.32 |
| ADI-55144-2 | 15.04 |
| ADI-55147 | 4.492 |
| ADI-55136 | 4.828 |
| ADI-55140 | 8.525 |
| CP-870893 | 7.283 |
| ADI-55145 | 6.662 |
| ADI-47720 | 5.544 |
| ADI-55164 | 8.056 |
| ADI-55168 | 8.848 |
| ADI-47741 | 17.86 |

### Example 6: Activation of Human CD40 Signaling Pathway by Progeny Anti-CD40 Antibodies

The activity of the parental and progeny anti-CD40 antibodies in activating the CD40 signaling with or without crosslinking by the F(ab)2 fragment of goat anti-human IgG-Fc (Jackson, catalog number: 109-036-098), was detected using a NFkB-Luc2P reporter gene assay.

Specifically, Jurkat cells overexpressing human CD40 were transfectd with the NFkB-Luc2P vector (Promega, catalog number: 11501ES03) and followed by applying pressure selection, CD40-NFkB-Jurkat cells were generated. Parental and progeny antibodies were added to CD40-NFkB-Jurkat cells at a final concentration of 200 nM with a 3-fold dilution across 11 concentrations, along with the F(ab)2 fragment of goat anti-human IgG-Fc (Thermo Scientific, catalog number: 88847) at a 1:2 molar ratio, and incubated in a cell culture incubator for 6 hours to investigate the CD40 downstream signaling activation induced by the antibody with the presence of anti-Fc fragment crosslinking. Additionally, antibodies at corresponding concentrations were added to CD40-NFkB-Jurkat cells and incubated in a cell culture incubator for 6 hours to examine the activation of the CD40 downstream signaling pathway induced by the antibodies alone, without anti-Fc fragment crosslinking.

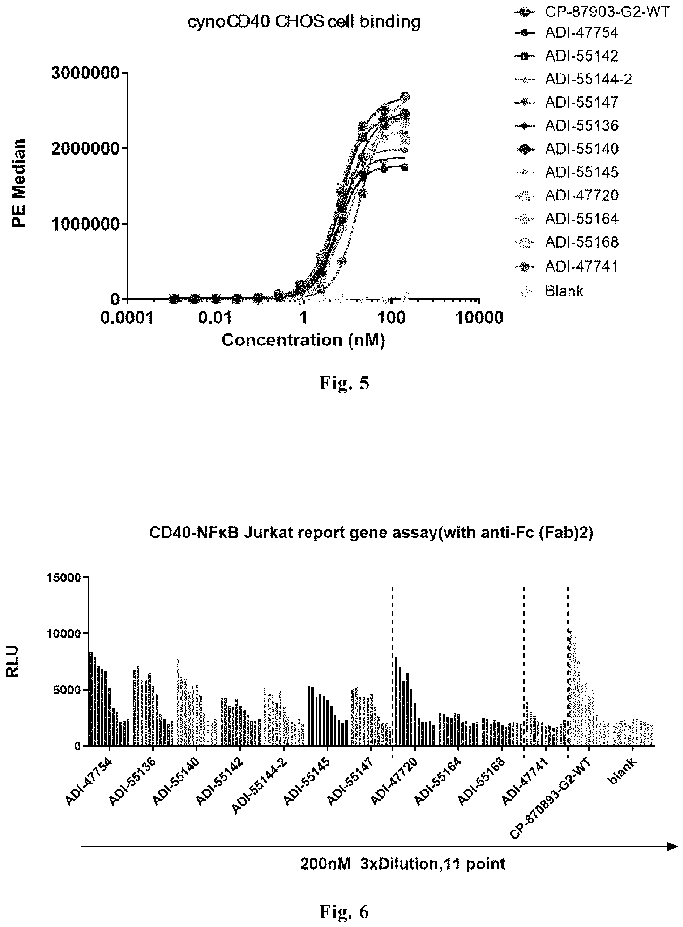

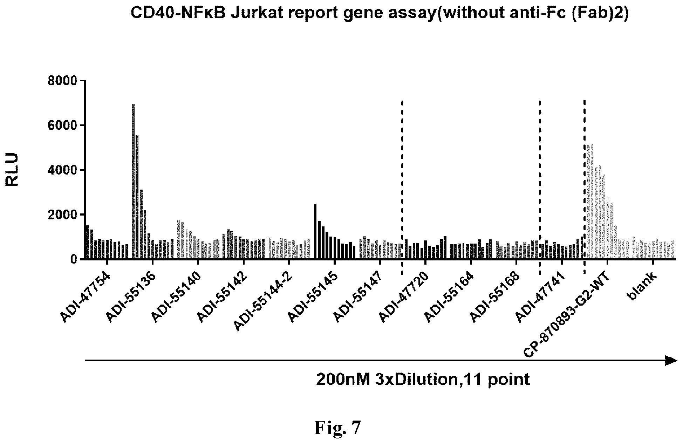

The experimental results are shown in Figs. 6 and 7. From the results, it can be seen that the parental antibodies ADI-47720, ADI-47741, ADI-47754, and the progeny antibodies ADI-55142, ADI-55144-2, ADI-55147, ADI-55164, ADI-55168 cannot activate the CD40 downstream signaling pathway without anti-Fc crosslinking, but can activate the CD40 downstream signaling pathway to varying degrees with anti-Fc crosslinking. The positive control antibody CP-870893-G2-WT can activate the CD40 downstream signaling pathway independently of anti-Fc crosslinking. Additionally, the control antibody CP-870893-G2-WT, as a crosslinking independent CD40 agonist, has been reported to cause severe hepatotoxicity in clinical settings, and its clinical trials have been halted.

Although the specific embodiments of the present application have been described in detail, those skilled in the art will understand that various modifications and changes can be made to the details based on all the teachings that have been disclosed, and these changes are all within the scope of protection of the present application. The entirety of the present application is given by the appended claims and any equivalents thereof.

## Claims

1. An antibody or antigen-binding fragment thereof capable of specifically binding to CD40, wherein the antibody or antigen-binding fragment thereof comprises:
(1) the following three complementary determining regions (CDR) of a heavy chain variable region (VH):
(a) a VH CDR1, having a structure selected from the group consisting of:
FTFX₁X₂YX₃MH (SEQ ID NO: 76), GSISSYYWS (SEQ ID NO: 6);
(b) a VH CDR2, having a structure selected from the group consisting of:
VIX₄YX₅X₆X₇X₈KYYAX₉SVKG (SEQ ID NO: 77), GISWSSX₁₀SIX₁₁YADSVKG (SEQ ID NO: 78), SIYYSGSTNYNPSLKS (SEQ ID NO: 22);
(c) a VH CDR3, having a structure selected from the group consisting of:
ARDTSRGHDI (SEQ ID NO: 23), AKDPTATTGARGYFDL (SEQ ID NO: 24), ARDANYYKWHPY (SEQ ID NO: 25);
and/or,
(2) the following three complementary determining regions (CDR) of a light chain variable region (VL):
(d) a VL CDR1, having a structure selected from the group consisting of:
X₁₂ASQSVSX₁₃X₁₄YLA (SEQ ID NO: 79), X₁₅ASX₁₆X₁₇ISSWLA (SEQ ID NO: 7);
(e) a VL CDR2, having a structure selected from the group consisting of:
GASX₁₈X₁₉X₂₀X₂₁ (SEQ ID NO: 8), AASX₂₂LX₂₃S (SEQ ID NO: 9);
(f) a VL CDR3, having a structure selected from the group consisting of:
X₂₄X₂₅YX₂₆DYPPFT (SEQ ID NO: 10), QQX₂₇X₂₈SX₂₉PPT (SEQ ID NO: 11);
wherein,
X₁ is selected from the group consisting of (i) amino acid residues S, E, G, D and (ii) amino acid residues that are conservative substitutions relative to (i);
X₂ is selected from the group consisting of (i) amino acid residues S, K, D, T and (ii) amino acid residues that are conservative substitutions relative to (i);
X₃ is selected from the group consisting of (i) amino acid residues G, A and (ii) amino acid residues that are conservative substitutions relative to (i);
X₄ is selected from the group consisting of (i) amino acid residues S, H and (ii) amino acid residues that are conservative substitutions relative to (i);
X₅ is selected from the group consisting of (i) amino acid residues E, H and (ii) amino acid residues that are conservative substitutions relative to (i);
X₆ is selected from the group consisting of (i) amino acid residues G, A and (ii) amino acid residues that are conservative substitutions relative to (i);
X₇ is selected from the group consisting of (i) amino acid residues S, E, T, V and (ii) amino acid residues that are conservative substitutions relative to (i);
X₈ is selected from the group consisting of (i) amino acid residues N, S, I, K and (ii) amino acid residues that are conservative substitutions relative to (i);
X₉ is selected from the group consisting of (i) amino acid residues D, E and (ii) amino acid residues that are conservative substitutions relative to (i);
X₁₀ is selected from the group consisting of (i) amino acid residues G, D and (ii) amino acid residues that are conservative substitutions relative to (i);
X₁₁ is selected from the group consisting of (i) amino acid residues G, H and (ii) amino acid residues that are conservative substitutions relative to (i);
X₁₂ is selected from the group consisting of (i) amino acid residues R, Q and (ii) amino acid residues that are conservative substitutions relative to (i);
X₁₃ is selected from the group consisting of (i) amino acid residues Y, S and (ii) amino acid residues that are conservative substitutions relative to (i);
X₁₄ is selected from the group consisting of (i) amino acid residues S, D, N and (ii) amino acid residues that are conservative substitutions relative to (i);
X₁₅ is selected from the group consisting of (i) amino acid residues R, E and (ii) amino acid residues that are conservative substitutions relative to (i);
X₁₆ is selected from the group consisting of (i) amino acid residues Q, K and (ii) amino acid residues that are conservative substitutions relative to (i);
X₁₇ is selected from the group consisting of (i) amino acid residues G, V and (ii) amino acid residues that are conservative substitutions relative to (i);
X₁₈ is selected from the group consisting of (i) amino acid residues S, T, A and (ii) amino acid residues that are conservative substitutions relative to (i);
X₁₉ is selected from the group consisting of (i) amino acid residues R, L and (ii) amino acid residues that are conservative substitutions relative to (i);
X₂₀ is selected from the group consisting of (i) amino acid residues N, A and (ii) amino acid residues that are conservative substitutions relative to (i);
X₂₁ is selected from the group consisting of (i) amino acid residues T, N and (ii) amino acid residues that are conservative substitutions relative to (i);
X₂₂ is selected from the group consisting of (i) amino acid residues S. Y and (ii) amino acid residues that are conservative substitutions relative to (i);
X₂₃ is selected from the group consisting of (i) amino acid residues E, Q and (ii) amino acid residues that are conservative substitutions relative to (i);
X₂₄ is selected from the group consisting of (i) amino acid residues S, G, Q and (ii) amino acid residues that are conservative substitutions relative to (i);
X₂₅ is selected from the group consisting of (i) amino acid residues E, Q, D and (ii) amino acid residues that are conservative substitutions relative to (i);
X₂₆ is selected from the group consisting of (i) amino acid residues A, S and (ii) amino acid residues that are conservative substitutions relative to (i);
X₂₇ is selected from the group consisting of (i) amino acid residues R, H, V and (ii) amino acid residues that are conservative substitutions relative to (i);
X₂₈ is selected from the group consisting of (i) amino acid residues S, L, A and (ii) amino acid residues that are conservative substitutions relative to (i);
X₂₉ is selected from the group consisting of (i) amino acid residues F. Y and (ii) amino acid residues that are conservative substitutions relative to (i).

2. The antibody or an antigen-binding fragment thereof according to claim 1, wherein the antibody or antigen-binding fragment thereof comprises:
(1) the following three complementary determining regions (CDR) of a heavy chain variable region (VH):
(a) a VH CDR1, which has a structure as shown in FTFX₁X₂YX₃MH (SEQ ID NO: 76);
(b) a VH CDR2, which has a structure as as shown in VIX₄YX₅X₆X₇X₈KYYAX₉SVKG (SEQ ID NO: 77);
(c) a VH CDR3, which has a structure as shown in ARDTSRGHDI (SEQ ID NO: 23); and/or,
(2) the following three complementary determining regions (CDR) of a light chain variable region (VL):
(d) a VL CDR1, which has a structure as shown in X₁₂ASQSVSX₁₃X₁₄YLA (SEQ ID NO: 79);
(e) a VL CDR2, which has a structure as shown in GASX₁₈X₁₉X₂₀X₂₁ (SEQ ID NO: 8);
(f) a VL CDR3, which has a structure as shown in X₂₄X₂₅YX₂₆DYPPFT (SEQ ID NO: 10); wherein,
X₁ is selected from the group consisting of (i) amino acid residues S, E, G and (ii) amino acid residues that are conservative substitutions relative to (i);
X₂ is selected from the group consisting of (i) amino acid residues S, K and (ii) amino acid residues that are conservative substitutions relative to (i);
X₃ is selected from the group consisting of (i) amino acid residue G and (ii) amino acid residues that are conservative substitutions relative to (i);
X₄ is selected from the group consisting of (i) amino acid residues S, H and (ii) amino acid residues that are conservative substitutions relative to (i);
X₅ is selected from the group consisting of (i) amino acid residues E, H and (ii) amino acid residues that are conservative substitutions relative to (i);
X₆ is selected from the group consisting of (i) amino acid residues G, A and (ii) amino acid residues that are conservative substitutions relative to (i);
X₇ is selected from the group consisting of (i) amino acid residues S, E, T, V and (ii) amino acid residues that are conservative substitutions relative to (i);
X₈ is selected from the group consisting of (i) amino acid residues N, S, I, K and (ii) amino acid residues that are conservative substitutions relative to (i);
X₉ is selected from the group consisting of (i) amino acid residues D, E and (ii) amino acid residues that are conservative substitutions relative to (i);
X₁₂ is selected from the group consisting of (i) amino acid residues R, Q and (ii) amino acid residues that are conservative substitutions relative to (i);
X₁₃ is selected from the group consisting of (i) amino acid residues Y, S and (ii) amino acid residues that are conservative substitutions relative to (i);
X₁₄ is selected from the group consisting of (i) amino acid residues S, D, N and (ii) amino acid residues that are conservative substitutions relative to (i);
X₁₈ is selected from the group consisting of (i) amino acid residues S, T, A and (ii) amino acid residues that are conservative substitutions relative to (i);
X₁₉ is selected from the group consisting of (i) amino acid residues R, L and (ii) amino acid residues that are conservative substitutions relative to (i);
X₂₀ is selected from the group consisting of (i) amino acid residues N, A and (ii) amino acid residues that are conservative substitutions relative to (i);
X₂₁ is selected from the group consisting of (i) amino acid residues T, N and (ii) amino acid residues that are conservative substitutions relative to (i);
X₂₄ is selected from the group consisting of (i) amino acid residues S, G, Q and (ii) amino acid residues that are conservative substitutions relative to (i);
X₂₅ is selected from the group consisting of (i) amino acid residues E, Q, D and (ii) amino acid residues that are conservative substitutions relative to (i);
X₂₆ is selected from the group consisting of (i) amino acid residues A, S and (ii) amino acid residues that are conservative substitutions relative to (i);
preferably, X₁ is selected from the group consisting of amino acid residues S, E, G; X₂ is selected from the group consisting of amino acid residues S, K; X₃ is amino acid residue G; X₄ is selected from the group consisting of amino acid residues S, H; X₅ is selected from the group consisting of amino acid residues E, H; X₆ is selected from the group consisting of amino acid residues G, A; X₇ is selected from the group consisting of amino acid residues S, E, T, V; X₈ is selected from the group consisting of amino acid residues N, S, I, K; X₉ is selected from the group consisting of amino acid residues D, E; X₁₂ is selected from the group consisting of amino acid residues R, Q; X₁₃ is selected from the group consisting of amino acid residues Y, S; X₁₄ is selected from the group consisting of amino acid residues S, D, N; X₁₈ is selected from the group consisting of amino acid residues S, T, A; X₁₉ is selected from the group consisting of amino acid residues R, L; X₂₀ is selected from the group consisting of amino acid residues N, A; X₂₁ is selected from the group consisting of amino acid residues T, N; X₂₄ is selected from the group consisting of amino acid residues S, G, Q; X₂₅ is selected from the group consisting of amino acid residues E, Q, D; X₂₆ is selected from the group consisting of amino acid residues A, S.

3. The antibody or an antigen-binding fragment thereof according to claim 2, compriseing:
a VH CDR1 as shown in any one of SEQ ID NOs: 1-3; a VH CDR2 as shown in any one of SEQ ID NOs: 12-18, 82; and, a VH CDR3 as shown in SEQ ID NO: 23; a VL CDR1 as shown in any one of SEQ ID NOs: 37-40; a VL CDR2 as shown in any one of SEQ ID NOs: 45-50; and, a VL CDR3 as shown in any one of SEQ ID NOs: 54-57;
preferably, wherein the antibody or antigen-binding fragment thereof comprises:
(1) a VH CDR1 as shown in SEQ ID NO: 1; a VH CDR2 as shown in SEQ ID NO: 12; and, a VH CDR3 as shown in SEQ ID NO: 23; a VL CDR1 as shown in SEQ ID NO: 37; a VL CDR2 as shown in SEQ ID NO: 45; and, a VL CDR3 as shown in SEQ ID NO: 54;
(2) a VH CDR1 as shown in SEQ ID NO: 1; a VH CDR2 as shown in SEQ ID NO: 13; and, a VH CDR3 as shown in SEQ ID NO: 23; a VL CDR1 as shown in SEQ ID NO: 37; a VL CDR2 as shown in SEQ ID NO: 45; and, a VL CDR3 as shown in SEQ ID NO: 54;
(3) a VH CDR1 as shown in SEQ ID NO: 2; a VH CDR2 as shown in SEQ ID NO: 14; and, a VH CDR3 as shown in SEQ ID NO: 23; a VL CDR1 as shown in SEQ ID NO: 38; a VL CDR2 as shown in SEQ ID NO: 46; and, a VL CDR3 as shown in SEQ ID NO: 55;
(4) a VH CDR1 as shown in SEQ ID NO: 2; a VH CDR2 as shown in SEQ ID NO: 15; and, a VH CDR3 as shown in SEQ ID NO: 23; a VL CDR1 as shown in SEQ ID NO: 39; a VL CDR2 as shown in SEQ ID NO: 47; and, a VL CDR3 as shown in SEQ ID NO: 56;
(5) a VH CDR1 as shown in SEQ ID NO: 2; a VH CDR2 as shown in SEQ ID NO: 16; and, a VH CDR3 as shown in SEQ ID NO: 23; a VL CDR1 as shown in SEQ ID NO: 39; a VL CDR2 as shown in SEQ ID NO: 48; and, a VL CDR3 as shown in SEQ ID NO: 57;
(6) a VH CDR1 as shown in SEQ ID NO: 2; a VH CDR2 as shown in SEQ ID NO: 17; and, a VH CDR3 as shown in SEQ ID NO: 23; a VL CDR1 as shown in SEQ ID NO: 40; a VL CDR2 as shown in SEQ ID NO: 49; and, a VL CDR3 as shown in SEQ ID NO: 57;
(7) a VH CDR1 as shown in SEQ ID NO: 3; a VH CDR2 as shown in SEQ ID NO: 18; and, a VH CDR3 as shown in SEQ ID NO: 23; a VL CDR1 as shown in SEQ ID NO: 38; a VL CDR2 as shown in SEQ ID NO: 50; and, a VL CDR3 as shown in SEQ ID NO: 54; or
(8) a VH CDR1 as shown in SEQ ID NO: 2; a VH CDR2 as shown in SEQ ID NO: 82; and, a VH CDR3 as shown in SEQ ID NO: 23; a VL CDR1 as shown in SEQ ID NO: 39; a VL CDR2 as shown in SEQ ID NO: 48; and, a VL CDR3 as shown in SEQ ID NO: 57.

4. The antibody or an antigen-binding fragment thereof according to claim 1, wherein the antibody or antigen-binding fragment thereof comprises:
(1) the following three complementary determining regions (CDR) of a heavy chain variable region (VH):
(a) a VH CDR1, which has a structure as shown in FTFX₁X₂YX₃MH (SEQ ID NO: 76);
(b) a VH CDR2, which has a structure as shown in GISWSSX₁₀SIX₁₁YADSVKG (SEQ ID NO: 78);
(c) a VH CDR3, which has a structure as shown in AKDPTATTGARGYFDL (SEQ ID NO: 24);
and/or,
(2) the following three complementary determining regions (CDR) of a light chain variable region (VL):
(d) a VL CDR1, which has a structure as shown in X₁₅ASX₁₆X₁₇ISSWLA (SEQ ID NO: 7);
(e) a VL CDR2, which has a structure as shown in AASX₂₂LX₂₃S (SEQ ID NO: 9);
(f) a VL CDR3, which has a structure as shown in QQX₂₇X₂₈SX₂₉PPT (SEQ ID NO: 11);
wherein,
X₁ is selected from the group consisting of (i) amino acid residue D and (ii) amino acid residues that are conservative substitutions relative to (i);
X₂ is selected from the group consisting of (i) amino acid residues D, T and (ii) amino acid residues that are conservative substitutions relative to (i);
X₃ is selected from the group consisting of (i) amino acid residue A and (ii) amino acid residues that are conservative substitutions relative to (i);
X₁₀ is selected from the group consisting of (i) amino acid residues G, D and (ii) amino acid residues that are conservative substitutions relative to (i);
X₁₁ is selected from the group consisting of (i) amino acid residues G, H and (ii) amino acid residues that are conservative substitutions relative to (i);
X₁₅ is selected from the group consisting of (i) amino acid residues R, E and (ii) amino acid residues that are conservative substitutions relative to (i);
X₁₆ is selected from the group consisting of (i) amino acid residues Q, K and (ii) amino acid residues that are conservative substitutions relative to (i);
X₁₇ is selected from the group consisting of (i) amino acid residues G, V and (ii) amino acid residues that are conservative substitutions relative to (i);
X₂₂ is selected from the group consisting of (i) amino acid residues S. Y and (ii) amino acid residues that are conservative substitutions relative to (i);
X₂₃ is selected from the group consisting of (i) amino acid residues E, Q and (ii) amino acid residues that are conservative substitutions relative to (i);
X₂₇ is selected from the group consisting of (i) amino acid residues R, H and (ii) amino acid residues that are conservative substitutions relative to (i);
X₂₈ is selected from the group consisting of (i) amino acid residues S, A and (ii) amino acid residues that are conservative substitutions relative to (i);
X₂₉ is selected from the group consisting of (i) amino acid residue F and (ii) amino acid residues that are conservative substitutions relative to (i);
preferably, X₁ is amino acid residue D; X₂ is selected from the group consisting of amino acid residues D, T; X₃ is amino acid residue A; X₁₀ is selected from amino acid residues G, D; X₁₁ is selected from the group consisting of amino acid residues G, H; X₁₅ is selected from the group consisting of amino acid residues R, E; X₁₆ is selected from the group consisting of amino acid residues Q, K; X₁₇ is selected from the group consisting of amino acid residues G, V; X₂₂ is selected from the group consisting of amino acid residues S, Y; X₂₃ is selected from the group consisting of amino acid residues E, Q; X₂₇ is selected from the group consisting of amino acid residues R, H; X₂₈ is selected from the group consisting of amino acid residues S, A; X₂₉ is amino acid residue F.

5. The antibody or an antigen-binding fragment thereof according to claim 4, comprising:
a VH CDR1 as shown in SEQ ID NOs: 4 or 5; a VH CDR2 as shown in any one of SEQ ID NOs: 19-21; and a VH CDR3 as shown in SEQ ID NO: 24; a VL CDR1 as shown in any one of SEQ ID NOs: 41-43; a VL CDR2 as shown in any one of SEQ ID NOs: 51-53 ; and a VL CDR3 as shown in SEQ ID NOs: 58 or 59;
preferably, the antibody or antigen-binding fragment thereof comprises:
(1) a VH CDR1 as shown in SEQ ID NO: 4; a VH CDR2 as shown in SEQ ID NO: 19; and, a VH CDR3 as shown in SEQ ID NO: 24; a VL CDR1 as shown in SEQ ID NO: 41; a VL CDR2 as shown in SEQ ID NO: 51; and a VL CDR3 as shown in SEQ ID NO: 58;
(2) a VH CDR1 as shown in SEQ ID NO: 4; a VH CDR2 as shown in SEQ ID NO: 20; and, a VH CDR3 as shown in SEQ ID NO: 24; a VL CDR1 as shown in SEQ ID NO: 42; a VL CDR2 as shown in SEQ ID NO: 52; and a VL CDR3 as shown in SEQ ID NO: 59; or
(3) a VH CDR1 as shown in SEQ ID NO: 5; a VH CDR2 as shown in SEQ ID NO: 21; and, a VH CDR3 as shown in SEQ ID NO: 24; a VL CDR1 as shown in SEQ ID NO: 43; a VL CDR2 as shown in SEQ ID NO: 53; and, a VL CDR3 as shown in SEQ ID NO: 59.

6. The antibody or an antigen-binding fragment thereof according to claim 1, comprising:
a VH CDR1 as shown in SEQ ID NO: 6; a VH CDR2 as shown in SEQ ID NO: 22; and, a VH CDR3 as shown in SEQ ID NO: 25; a VL CDR1 as shown in SEQ ID NO: 44; a VL CDR2 as shown in SEQ ID NO: 45; and a VL CDR3 as shown in SEQ ID NO: 60 .

7. The antibody or antigen-binding fragment thereof according to any one of claims 1 to 6, wherein the antibody or antigen-binding fragment thereof further comprises a framework region of human immunoglobulin;
for example, wherein the antibody or antigen-binding fragment thereof comprises a framework region contained in an amino acid sequence encoded by a human germline antibody gene; for example, wherein the antibody or antigen-binding fragment thereof comprises: a heavy chain framework region contained in an amino acid sequence encoded by a human heavy chain germline gene, and/or a light chain framework region contained in an amino acid sequence encoded by a human light chain germline gene.

8. The antibody or an antigen-binding fragment thereof according to any one of claims 1 to 7, comprising:
(1) a heavy chain variable region (VH) comprising an amino acid sequence selected from the following:
(i) the sequence as shown in any one of SEQ ID NOs:26-36, 83;
(ii) a sequence having a substitution, deletion, or addition of one or several amino acids (e.g., a substitution, deletion or addition of 1, 2, 3, 4 or 5 amino acids) as compared to the sequence shown in any one of SEQ ID NOs: 26-36, 83; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 26-36, 83;
and/or,
(2) a light chain variable region (VL) comprising an amino acid sequence selected from the following:
(i) the sequence as shown in any one of SEQ ID NOs:61-70;
(ii) a sequence having a substitution, deletion, or addition of one or several amino acids (e.g., a substitution, deletion or addition of 1, 2, 3, 4 or 5 amino acids) as compared to the sequence shown in any one of SEQ ID NOs: 61-70; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 61-70;
preferably, wherein the antibody or antigen-binding fragment thereof comprises:
(a) a VH comprising the sequence as shown in any one of SEQ ID NOs: 26-32, 83 or variant thereof, and a VL comprising the sequence as shown in any one of SEQ ID NOs: 61-66 or variant thereof;
or,
(b) a VH comprising the sequence as shown in any one of SEQ ID NOs: 33-35 or variant thereof, and a VL comprising the sequence as shown in any one of SEQ ID NOs: 67-69 or variant thereof;
wherein, the variant has a substitution, deletion, or addition of one or several amino acids (e.g., a substitution, deletion or addition of 1, 2, 3, 4 or 5 amino acids), or has a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution;
for example, the antibody or antigen-binding fragment thereof comprises a VH of the sequence shown in SEQ ID NO: 26 or variant thereof, and a VL of the sequence shown in SEQ ID NO: 61 or variant thereof;
for example, the antibody or antigen-binding fragment thereof comprises a VH of the sequence shown in SEQ ID NO: 27 or variant thereof, and a VL of the sequence shown in SEQ ID NO: 61 or variant thereof;
for example, the antibody or antigen-binding fragment thereof comprises a VH of the sequence shown in SEQ ID NO: 28 or variant thereof, and a VL of the sequence shown in SEQ ID NO: 62 or variant thereof;
for example, the antibody or antigen-binding fragment thereof comprises a VH of the sequence shown in SEQ ID NO: 29 or variant thereof, and a VL of the sequence shown in SEQ ID NO: 63 or variant thereof;
for example, the antibody or antigen-binding fragment thereof comprises a VH of the sequence shown in SEQ ID NO: 30 or variant thereof, and a VL of the sequence shown in SEQ ID NO: 64 or variant thereof;
for example, the antibody or antigen-binding fragment thereof comprises a VH of the sequence shown in SEQ ID NO: 31 or variant thereof, and a VL of the sequence shown in SEQ ID NO:65 or variant thereof;
for example, the antibody or antigen-binding fragment thereof comprises a VH of the sequence shown in SEQ ID NO: 32 or variant thereof, and a VL of the sequence shown in SEQ ID NO: 66 or variant thereof;
for example, the antibody or antigen-binding fragment thereof comprises a VH of the sequence shown in SEQ ID NO: 33 or variant thereof, and a VL of the sequence shown in SEQ ID NO: 67 or variant thereof;
for example, the antibody or antigen-binding fragment thereof comprises a VH of the sequence shown in SEQ ID NO: 34 or variant thereof, and, a VL of the sequence shown in SEQ ID NO: 68 or variant thereof;
for example, the antibody or antigen-binding fragment thereof comprises a VH of the sequence shown in SEQ ID NO: 35 or variant thereof, and a VL of the sequence shown in SEQ ID NO: 69 or variant thereof;
for example, the antibody or antigen-binding fragment thereof comprises a VH of the sequence shown in SEQ ID NO: 36 or variant thereof, and a VL of the sequence shown in SEQ ID NO: 70 or variant thereof;
for example, the antibody or antigen-binding fragment thereof comprises a VH of the sequence shown in SEQ ID NO: 83 or variant thereof, and a VL of the sequence shown in SEQ ID NO: 64 or variant thereof;
wherein, the variant has a substitution, deletion, or addition of one or several amino acids (e.g., a substitution, deletion or addition of 1, 2, 3, 4 or 5 amino acids), or has a sequence identity of at least 80%, at least 85%, at least 90%, at least 91%, at least 92%, at least 93%, at least 94%, at least 95%, at least 96%, at least 97%, at least 98%, at least 99%, or 100% , as compared to the sequence from which it is derived; preferably, the substitution is a conservative substitution.

9. The antibody or antigen-binding fragment thereof according to any one of claims 1 to 8, which further comprises a constant region derived from a human immunoglobulin;
preferably, the heavy chain of the antibody or antigen-binding fragment thereof comprises a constant region of the heavy chain derived from a human immunoglobulin (e.g., IgG1, IgG2, IgG3 or IgG4);
preferably, the light chain of the antibody or antigen-binding fragment thereof comprises a light chain constant region (e.g., a constant region of κ or λ chain) derived from a human immunoglobulin.

10. The antibody or antigen-binding fragment thereof according to any one of claims 1 to 9, wherein the antigen-binding fragment is selected from the group consisting of Fab, Fab', F(ab')₂, Fv, disulfide-linked Fv, scFv, diabody, and single-domain antibody (sdAb); and/or, the antibody is a chimeric antibodies, bispecific antibodies or multispecific antibody.

11. An isolated nucleic acid molecule, which encodes the antibody or antigen-binding fragment thereof according to any one of claims 1 to 10, or its heavy chain variable region and/or light chain variable region.

12. A vector, which comprises the nucleic acid molecule according to claim 11; preferably, the vector is a cloning vector or an expression vector.

13. A host cell, which comprises the nucleic acid molecule according to claim 11 or the vector according to claim 12.

14. A method of preparing the antibody or antigen-binding fragment thereof according to any one of claims 1 to 10, comprising culturing the host cell according to claim 13 under a condition that allows the expression of the antibody or antigen-binding fragment thereof, and recovering the antibody or antigen-binding fragment thereof from a cell culture of host cell.

15. A pharmaceutical composition, which comprises the antibody or antigen-binding fragment thereof according to any one of claims 1 to 10, and a pharmaceutically acceptable carrier and/or excipient.

16. Use of an antibody or antigen-binding fragment thereof according to any one of claims 1 to 10, the isolated nucleic acid molecule according to claim 11, the vector according to claim 12, or the host cell according to claim 13 in the manufacture of a medicament, wherein the medicament is used for activating CD40, increasing an immune cell activity, enhancing an immune response, and/or preventing and/or treating a tumor or an infection in a subject;
preferably, the immune cells is a T cell, a B cell, a DC, a macrophage and/or a NK cell;
preferably, the immune response is a CD40-mediated immune response;
preferably, the subject is a mammal, such as a human;
preferably, the antibody or antigen-binding fragment thereof is used alone or in combination with an additional pharmaceutically active agent.

17. A method for enhancing an immune response, and/or preventing and/or treating a tumor or infection in a subject; the method comprising: administering to a subject in need thereof an effective amount of the antibody or antigen-binding fragment thereof according to any one of claims 1 to 10, or the pharmaceutical composition according to claim 15;
preferably, the immune response is a CD40-mediated immune response;
preferably, the subject is a mammal, such as a human.

18. A conjugate, which comprises the antibody or antigen-binding fragment thereof according to any one of claims 1 to 10, and a detectable label connected to the antibody or antigen-binding fragment thereof;
preferably, the detectable label is selected from enzymes (e.g., horseradish peroxidase or alkaline phosphatase), chemiluminescent reagents (e.g., acridine ester compounds, luminal and thereof, or ruthenium derivative), fluorescent dye (e.g., fluorescein or fluorescent protein), radionuclide or biotin.

19. A kit, which comprises the antibody or antigen-binding fragment thereof according to any one of claims 1 to 10 or the conjugate according to claim 18;
preferably, the kit comprises the conjugate acccording to claim 18;
preferably, the kit comprises the antibody or antigen-binding fragment thereof according to any one of claims 1 to 10, and a secondary antibody that specifically recognizes the antibody or antigen-binding fragment thereof; optionally, the secondary antibody further comprises a detectable label, such as an enzyme (e.g., horseradish peroxidase or alkaline phosphatase), chemiluminescent reagent (e.g., acridinium ester, luminal and derivative thereof, or ruthenium derivative), fluorescent dye (e.g., fluorescein or fluorescent protein), radionuclide or biotin.

20. A method for detecting the presence or level of CD40 in a sample, comprising using the antibody or antigen-binding fragment thereof according to any one of claims 1 to 10 or the conjugate acccording to claim 18;
preferably, the method is an immunological assay, such as an immunoblotting assay, an enzyme immunoassay (e.g. ELISA), a chemiluminescent immunoassay, a fluorescent immunoassay or a radioimmunoassay;
preferably, the method comprises using the conjugate according to claim 18;
preferably, the method comprises using the antibody or antigen-binding fragment according to any one of claims 1 to 10, and the method further comprises using a second antibody carrying a detectable label (e.g., an enzyme (e.g., horseradish peroxidase or alkaline phosphatase), a chemiluminescent reagent (e.g., an acridinium ester, luminol and derivative thereof, or ruthenium derivativ), a fluorescent dye (e.g., fluorescein or fluorescent protein), radionuclide, or biotin) to detect the antibody or antigen-binding fragment thereof.

21. Use of the antibody or antigen-binding fragment thereof according to any one of claims 1 to 10 or the conjugate according to claim 18 in the manufacture of a detection reagent, wherein the detection reagent is used to detect the presence or level of CD40 in a sample;
preferably, the detection reagent detects the presence or level of CD40 in the sample by the method according to claim 20;
preferably, the sample is a cell sample (e.g., an immune cell) from a subject (e.g., a mammal, preferably a human , or a cynomolgus monkey).
